# EUROPÄISCHE PATENTSCHRIFT

(11) **EP 0 655 435 B1**
(45) Veröffentlichungstag und Bekanntmachung des Hinweises auf die Patenterteilung: **15.07.1998**
(21) Anmeldenummer: 94810659.6
(22) Anmeldetag: 21.11.1994
(51) Int. Cl.: C07C 219/10, C07C 217/08, C07D 279/22, C08K 5/17, C08K 5/46, C10M 133/14, C10M 135/36

(54) **4-Hydroxyphenylalkylcarbonsäure(di)arylaminoalkylester und ihre Verwndung als Stabilisatoren organischen Materials**
4-Hydroxyphenylalkyl carboxylic acid (di)arylaminoalkyl esters and their use as stabilisers for organic material
Esters diarylaminoalkyl d'acide 4-hydroxyalkanoique et leur utilisation comme stabilisants dans des matériaux organiques

(30) Priorität: 29.11.1993 CH 3558/93
(43) Veröffentlichungstag der Anmeldung: 31.05.1995
(73) Patentinhaber: Ciba Specialty Chemicals Holding Inc., 4057 Basel (CH)
(72) Erfinder: Camenzind, Hugo, Dr., CH-3014 Bern (CH)

(56) Entgegenhaltungen:
- EP-A- 0 286 595
- DE-A- 3 426 367
- US-A- 3 457 286
- US-A- 3 873 459
- US-A- 3 920 729
- US-A- 4 448 969

## Beschreibung

Die vorliegende Erfindung betrifft neue, zur Stabilisierung organischen Materials geeignete Ester von 4-Hydroxyphenylcarbonsäuren, diese enthaltende Zusammensetzungen, ein Verfahren zu ihrer Herstellung sowie ihre Verwendung als Stabilisatoren.

Es ist bekannt, organische Stoffe mit Stabilisatoren zu versetzen, um deren Abbau durch elektromagnetische Wellen, Chemikalien oder Wärme zu verhindern. Man bedient sich dabei schon seit langem der sterisch gehinderten Phenole sowie der aromatischen Amine (Vgl. Ullmanns Encyclopedia of Industrial Chemistry, 5th Ed. Vol. A3 (1985) pp 91-111,"Antioxidants").

Man kennt inzwischen auch Antioxidantien, welche Amin- und Phenolfunktion in einem Molekül vereinigen (vgl. EP-A-0,119,160, US-A-3,225,099, -2,962,531, -3,119,871, -3,224,974, -3 457 286, -4 448 969 und -3 920 729).

In Schmiermitteln, z.B. Motorölen, können solche Antioxidantien ebenfalls eingesetzt werden. Dort kommt es besonders auf die Verhinderung von Schlammablagerungen (deposit control) an, welche die Lebensdauer von Verbrennungsmotoren erheblich herabsetzen können.

Es wurde nun überraschenderweise gefunden, daß Stoffe, die in bestimmter Weise verbundene phenolische und aminische Gruppen enthalten, sich besonders gut als Stabilisatoren, vor allem für Schmiermittel, eignen.

Die Erfindung betrifft daher Verbindungen der Formel I 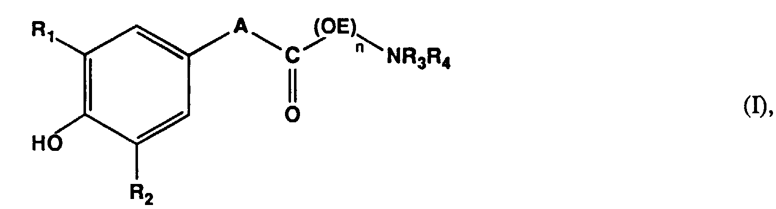 worin
- R₁ und R₂: unabhängig voneinander C₁-C₂₀-Alkyl, Allyl, Methallyl, unsubstituiertes oder mit C₁-C₈-Alkyl substituiertes C₅-C₁₂-Cycloalkyl, C₅-C₈-Cycloalkenyl, Phenyl oder C₇-C₉-Phenylalkyl bedeuten,
- A: für eine direkte Bindung, -CH₂-, -(CH₂)₂- oder -CH₂-CH(CH₃)- steht,
- E: -(CH₂)₂-, -CHR₆-CH₂- oder -CH₂CHR₆- darstellt,
- n: 1,2 oder 3 ist,
- R₃: C₁-C₁₈-Alkyl oder durch -O- oder -S- unterbrochenem C₂-C₁₈-Alkyl, unsubstituiertem oder mit C₁-C₈-Alkyl substituiertem C₅-C₁₂-Cycloalkyl, Phenyl, Naphthyl, C₇-C₉-Phenylalkyl, C₇-C₁₈-Alkylphenyl oder der Formel 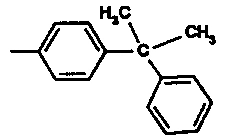 entspricht,
- R₄: Phenyl, Naphthyl, C₇-C₉-Phenylalkyl, C₇-C₁₈-Alkylphenyl oder ein Rest der Formel 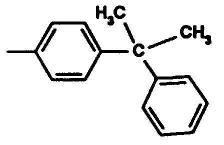 ist,
oder
- NR₃R₄: eine Gruppe der Formel 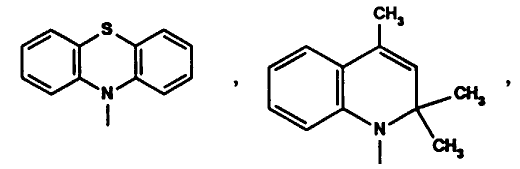 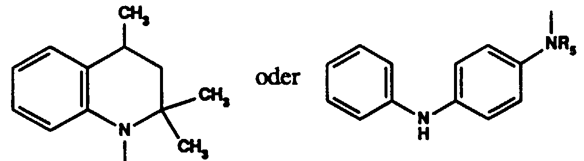 bedeutet,
- R₅: für Wasserstoff, C₁-C₁₈-Alkyl, Allyl, Methallyl, unsubstituiertes oder mit C₁-C₈-Alkyl substituiertes C₅-C₁₂-Cycloalkyl, Phenyl oder Naphthyl steht,
- R₆: Wasserstoff, C₁-C₂₀-Alkyl oder durch -O-, -S-, -NR₇- oder -C(O)O- unterbrochenes C₂-C₂₀-Alkyl ist, und
- R₇: Wasserstoff oder C₁-C₆-Alkyl darstellt.

R₁, R₂, R₃, R₅, und R₆ in der Bedeutung von C₁-C₁₈Alkyl bzw. C₁-C₂₀Alkyl können gerad- oder verzweigtkettig sein und sind beispielsweise Methyl, Ethyl, Propyl, Isopropyl, n-Butyl, Isobutyl, t-Butyl, Pentyl, Isopentyl, Hexyl, Heptyl, Octyl, 2-Ethylhexyl, Nonyl, Decyl, Undecyl, Dodecyl, Tridecyl, Tetradecyl, Pentadecyl, Hexadecyl, Heptadecyl, Octadecyl, 2-Ethylbutyl, 1-Methylpentyl, 1,3-Dimethylbutyl, 1,1,3,3-Tetramethylbutyl, 1-Methylhexyl, Isoheptyl, 1-Methylheptyl, 1,1,3-Trimethylhexyl, 1-Methylundecyl bzw. zusätzlich noch beispielsweise Eicosyl. Bedeuten diese Reste Gruppen mit einer geringeren Zahl von C-Atomen, so sind entsprechende Beispiele ebenfalls obiger Liste zu entnehmen.
Dies gilt auch für R₇ und das weiter unten bei Formel III eingeführte R₈. Die genannten Alkylreste haben bevorzugt 1-12, insbesondere 1-6, vor allem 1-4 C-Atome.

Stellen R₁, R₂, R₄ und R₃ C₇-C₉-Phenylalkyl dar, so handelt es sich beispielsweise um Benzyl, 1- oder 2-Phenylethyl, 3-Phenylpropyl, α,α-Dimethylbenzyl oder 2-Phenylisopropyl, vorzugsweise jedoch um Benzyl.

R₄ und R₃ als C₇-C₁₈-Alkylphenyl können lineare oder verzweigte Alkylgruppen enthalten, wobei zweckmäßig 1-3, insbesondere 1 oder 2 Alkylgruppen vorhanden sind. Beispiele sind Tolyl, die isomeren Xylyle, Ethylphenyl, Isopropylphenyl, tert-Butylphenyl, sec-Pentylphenyl, n-Hexylphenyl, tert-Octylphenyl, iso-Nonylphenyl oder n-Dodecylphenyl.

Beispiele für die obengenannten unsubstituierten oder C₁-C₈-alkylsubstituierten Cycloalkylgruppen mit 5-12 C-Atomen (bevorzugt sind solche mit 5-8, insbesondere 5 oder 6 C-Atomen) sind Cyclopentyl, Cyclohexyl, Cyclooctyl, Cyclododecyl, 2- oder 4-Methylcyclohexyl, Dimethylcyclohexyl, Trimethylcyclohexyl, t-Butylcyclohexyl, wobei Cyclohexyl besonders bevorzugt ist.

C₅-C₈-Cycloalkenylreste unterscheiden sich von den oben beschriebenen Clycloalkylresten nur durch eine >C=C< Doppelbindung im Ring. Als C₅-C₈-Cycloalkenyl bedeuten R₁ und R₂ bevorzugt Cyclopentenyl und -hexenyl.

Bei den oben genannten unterbrochenen Alkylresten (R₃ und R₆) ist das unterbrechende Atom oder die unterbrechende Gruppe innerhalb der C-Kette angesiedelt, die ein- oder mehrfach unterbrochen sein kann. Bevorzugt ist die Unterbrechung durch nur ein Mitglied der Gruppe -O-,-S-, -NR₇- oder -CO-O-. Sind R₃ und R₆ durch Sauerstoff oder Schwefel unterbrochen, dann enthalten sie z.B. Struktureinheiten wie -CH₂CH₂-O-CH₂CH₂-, -CH₂CH₂-S-CH₂CH₂-, oder -O-(CH₂)₆-O-. Die Einheiten (O-CH₂-CH₂) und (S-CH₂CH₂) können insbesondere auch mehrfach nebeneinander auftreten. Beispiele für durch -O- oder -S- unterbrochen Reste sind CH₃-O-CH₂-, CH₃-S-CH₂-, CH₃-O-CH₂CH₂-O-CH₂-, CH₃-(O-CH₂CH₂-)₂O-CH₂-, CH₃-(O-CH₂CH₂-)₃O-CH₂- oder CH₃-(O-CH₂CH₂-)₄O-CH₂-.
Durch >NR₇ unterbrochenes C₂-C₂₀-Alkyl bedeutet beispielsweise CH₃-NH-CH₂- oder CH₃-N(CH₃)-CH₂-.
Durch -CO-O- unterbrochenes C₂-C₂₀-Alkyl bedeutet beispielsweise CH₃-CO-O-CH₂- oder C₁-C₁₈alkyl-CO-OCH₂CH₂-.

Wenn R₆ als Alkyl keine solche Unterbrechung aufweist, so ist es vorzugsweise C₁-C₁₇-Alkyl, z.B. C₁-C₄-Alkyl, und besonders Methyl.

Gegenstand der Erfindung sind auch Mischungen von Verbindungen der Formel I mit verschiedenem Index n, besonders solche Mischungen, wie sie aus dem weiter unten beschriebenen Verfahren erhältlich sind.

Der Index n steht vorzugsweise für 1 oder 2, vor allem für 1.

Bevorzugte Verbindungen der Formel I sind solche, worin
- R₁ und R₂: unabhängig voneinander C₁-C₈-Alkyl, C₅-C₈-Cycloalkyl oder C₇-C₉-Phenylalkyl bedeuten,
- A: für -(CH₂)₂- oder -CH₂-CH(CH₃)- steht,
- E: -(CH₂)₂-, -CHR₆-CH₂- oder -CH₂CHR₆- darstellt,
- n: 1 oder 2 ist,
- R₃ und R₄: unabhängig voneinander
Phenyl, Naphthyl, C₇-C₉-Phenylalkyl, C₇-C₁₈-Alkylphenyl entsprechen, oder
- NR₃R₄: eine Gruppe der Formel 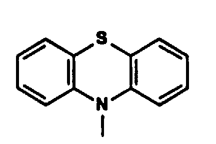 oder 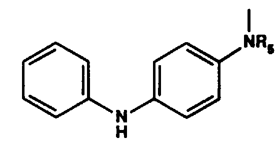 bedeutet,
- R₅: Wasserstoff, C₁-C₈-Alkyl oder Phenyl ist,
- R₆: Wasserstoff, C₁-C₂₀-Alkyl oder durch -O- oder -S- unterbrochenes C₂-C₂₀-Alkyl ist.

Besonders bevorzugt sind diejenigen Verbindungen der Formel I, worin R₁ und R₂ unabhängig voneinander C₁-C₅-Alkyl, C₅-C₆-Cycloalkyl oder C₇-C₉-Phenylalkyl bedeuten,
- n: 1 oder 2 ist,
- A: für -(CH₂)₂- oder -CH₂-CH(CH₃)- steht,
- E: -(CH₂)₂-, -CHR₆-CH₂- oder -CH₂CHR₆- darstellt,
- R₃ und R₄: unabhängig voneinander Phenyl, Naphthyl, C₇-C₉-Phenylalkyl, C₇-C₁₈-Alkylphenyl sind, oder
NR₃R₄ eine Gruppe der Formel 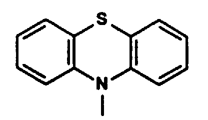 bedeutet und
- R₆: Wasserstoff oder C₁-C₄-Alkyl ist.

Die Verbindungen der Formel I eignen sich hervorragend zum Stabilisieren von organischen Materialien gegen lichtinduzierten, thermischen oder/und oxidativen Abbau. Gegenstand der Erfindung sind daher auch Zusammensetzungen enthaltend ein gegen solche Abbaureaktionen empfindliches organisches Material und mindestens eine Verbindung der Formel I bzw. die Verwendung von Verbindungen der Formel I als Stabilisatoren für organische Materialien gegen die genannten Abbauarten.

Die Verbindungen der Formel I können insbesondere als Stabilisatoren für natürliche, halbsynthetische oder synthetische Polymere, besonders thermoplastische Kunststoffe und Elastomere, sowie für funktionelle Flüssigkeiten, insbesondere Schmierstoffe und Hydraulikflüssigkeiten, eingesetzt werden. Beispiele für solche Substrate sind der folgenden Aufzählung von geeigneten Materialien zu entnehmen.
1. Polymere von Mono- und Diolefinen, beispielsweise Polypropylen, Polyisobutylen, Polybuten-1, Poly-4-methylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE), verzweigtes Poly-ethylen niederer Dichte (VLDPE).
   Polyolefine, d.h. Polymere von Monoolefinen, wie sie beispielhaft im vorstehenden Absatz erwähnt sind, insbesondere Polyethylen und Polypropylen, können nach verschiedenen Verfahren hergestellt werden, insbesondere nach den folgenden Methoden:
   a) radikalisch (gewöhnlich bei hohem Druck und hoher Temperatur).
   b) mittels Katalysator, wobei der Katalysator gewöhnlich ein oder mehrere Metalle der Gruppe IVb, Vb, VIb oder VIII enthält. Diese Metalle besitzen gewöhnlich einen oder mehrere Liganden wie Oxide, Halogenide, Alkoholate, Ester, Ether, Amine, Alkyle, Alkenyle und/oder Aryle, die entweder π- oder σ-koordiniert sein können. Diese Metallkomplexe können frei oder auf Träger fixiert sein, wie beispielsweise auf aktiviertem Magnesiumchlorid, Titan(III)chlorid, Aluminiumoxid oder Siliziumoxid. Diese Katalysatoren können im Polymerisationsmedium löslich oder unlöslich sein. Die Katalysatoren können als solche in der Polymerisation aktiv sein, oder es können weitere Aktivatoren verwendet werden, wie beispielsweise Metallalkyle, Metallhydride, Metallalkylhalogenide, Metallalkyloxide oder Metallalkyloxane, wobei die Metalle Elemente der Gruppen Ia, IIa und/oder IIIa sind. Die Aktivatoren können beipielsweise mit weiteren Ester-, Ether-, Amin- oder Silylether-Gruppen modifiziert sein. Diese Katalysatorsysteme werden gewöhnlich als Phillips, Standard Oil Indiana, Ziegler (-Natta), TNZ (DuPont), Metallocen oder Single Site Katalysatoren (SSC) bezeichnet.
2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).
3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere und deren Copolymere mit Kohlenstoffmonoxid, oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere, LLDPE/Ethylen-Acrylsäure-Copolymere und alternierend oder statistisch aufgebaute Polyalkylen/Kohlenstoffmonoxid-Copolymere und deren Mischungen mit anderen Polymeren wie z.B. Polyamiden.
4. Kohlenwasserstoffharze (z.B. C₅-C₉) inklusive hydrierte Modifikationen davon (z.B. Klebrigmacherharze) und Mischungen von Polyalkylenen und Stärke.
5. Polystyrol, Poly-(p-methylstyrol), Poly-(α-methylstyrol).
6. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Butadien-Alkylacrylat und -methacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.
7. Pfropfcopolymere von Styrol oder α-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 6) genannten Copolymeren, wie sie z.B. als sogenannte ABS-, MBS-, ASA- oder AES-Polymere bekannt sind.
8. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.
9. Polymere, die sich von α,β-ungesättigten Säuren und deren Derivaten ableiten, wie Polyacrylate und Polymethacrylate, mit Butylacrylat schlagzäh modifizierte Polymethylmethacrylate, Polyacrylamide und Polyacrylnitrile.
10. Copolymere der unter 9) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.
11. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.
12. Homo- und Copolymere von cyclischen Ethern, wie Polyalkylenglykole, Polyethylenoxyd, Polypropylenoxyd oder deren Copolymere mit Bisglycidylethern.
13. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.
14. Polyphenylenoxide und -sulfide und deren Mischungen mit Styrolpolymeren oder Polyamiden.
15. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.
16. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, 12/12, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid oder Poly-m-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").
17. Polyharnstoffe, Polyimide, Polyamid-imide und Polybenzimidazole.
18. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.
19. Polycarbonate und Polyestercarbonate.
20. Polysulfone, Polyethersulfone und Polyetherketone.
21. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.
22. Trocknende und nicht-trocknende Alkydharze.
23. Ungesättigte Polyesterharze, die sich von Copolyestern gsättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.
24. Vernetzbare Acrylharze, die sich von substituierten Acrylsäureestern ableiten, wie z.B. von Epoxyacrylaten, Urethan-acrylaten oder Polyester-acrylaten.
25. Alkydharze, Polyesterharze und Acrylatharze, die mit Melaminharzen, Harnstoffharzen, Polyisocyanaten oder Epoxidharzen vernetzt sind.
26. Vernetzte Epoxidharze, die sich von Polyepoxiden ableiten, z.B. von Bis-glycidylethern oder von cycloaliphatischen Diepoxiden.
27. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.
28. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.
29. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z.B. Phthalate, Adipate, Phosphate oder Trimellitate), sowie Abmischungen synthetischer Ester mit Mineralölen in beliebigen Gewichtsverhältnissen, wie sie z.B. als Spinnpräparationen Anwendung finden, sowie deren wässrige Emulsionen.
30. Wässrige Emulsionen natürlicher oder synthetischer Kautschuke, wie z.B. Naturkautschuk-Latex oder Latices von carboxylierten Styrol-Butadien-Copolymeren.

In den erfindungsgemäßen Zusammensetzungen sind die Verbindungen der Formel I zweckmäßig zu 0.01 bis 10, beispielsweise zu 0.05 bis 5, vorzugsweise zu 0.05 bis 3, insbesondere jedoch zu 0.1 bis 2 Gew.-% enthalten. Es kann sich dabei um eine oder mehrere dieser Verbindungen der Formel I handeln, und die Gewichtsprozentangaben beziehen sich auf die gesamte Menge dieser Verbindungen. Berechnungsgrundlage ist dabei das Gesamtgewicht des organischen Materials ohne die Verbindungen der Formel I.

Die Einarbeitung in die Materialien kann beispielsweise durch Einmischen oder Aufbringen der Verbindungen der Formel I und gegebenenfalls weiterer Additive nach den in der Technik üblichen Methoden erfolgen. Handelt es sich um Polymere, insbesondere synthetische Polymere, kann die Einarbeitung vor oder während der Formgebung, oder durch Aufbringen der gelösten oder dispergierten Verbindungen auf das Polymere, gegebenenfalls unter nachträglichem Verdunsten des Lösungsmittels erfolgen. Im Fall von Elastomeren können diese auch als Latices stabilisiert werden. Eine weitere Möglichkeit der Einarbeitung der Verbindungen der Formel I in Polymere besteht in deren Zugabe vor, während oder unmittelbar nach der Polymerisation der entsprechenden Monomeren bzw. vor der Vernetzung. Die Verbindungen der Formel I können dabei als solche, aber auch in encapsulierter Form (z.B in Wachsen, Ölen oder Polymeren) zugesetzt werden. Im Falle der Zugabe vor oder während der Polymerisation können die Verbindungen der Formel I auch als Regulatoren für die Kettenlänge der Polymeren (Kettenabbrecher) wirken.

Die Verbindungen der Formel I oder Mischungen davon können auch in Form eines Masterbatches, der diese Verbindungen beispielsweise in einer Konzentration von 2.5 bis 25 Gew.-% enthält, den zu stabilisierenden Kunststoffen zugesetzt werden.

Zweckmäßig kann die Einarbeitung der Verbindungen der Formel I nach folgenden Methoden erfolgen:
- als Emulsion oder Dispersion (z.B. zu Latices oder Emulsionspolymeren)
- Als Trockenmischung während des Vermischens von Zusatzkomponenten oder Polymermischungen
- durch direktes Zugeben in die Verarbeitungsapparatur (z.B. Extruder, Innenmischer usw.)
- als Lösung oder Schmelze.

Erfindungsgemäße Polymerzusammensetzungen können in verschiedener Form angewandt bzw. zu verschiedenen Produkten verarbeitet werden, z.B. als (zu) Folien, Fasern, Bändchen, Formmassen, Profilen, oder als Bindemittel für Lacke, Klebstoffe oder Kitte.

Die Erfindung betrifft auch ein Verfahren zum Stabilisieren von organischem Material, insbesondere von thermoplastischen Polymeren, Elastomeren oder funktionellen Flüssigkeiten, vor allem Schmierstoffen, gegen oxidativen, thermischen und/oder lichtinduzierten Abbau, dadurch gekennzeichnet, daß man diesem Material als Stabilisatoren Verbindungen der Formel I zusetzt bzw. auf dieses aufbringt.

Die Verbindungen der Formel I sind beispielsweise besonders geeignet, um funktionellen Flüssigkeiten verbesserte Gebrauchseigenschaften zu verleihen. Es ist dabei insbesondere auf ihre überraschend gute antioxidative und schlammverhindernde (deposit control) Wirkung hinzuweisen. Deshalb umfaßt die Erfindung auch Zusammensetzungen, enthaltend eine funktionelle Flüssigkeit und wenigstens eine Verbindung der allgemeinen Formel I, wie oben beschrieben.

Als funktionelle Flüssigkeiten sind beispielsweise Schmierstoffe, Hydraulikflüssigkeiten und Metallbearbeitungsflüssigkeiten zu nennen. Schmierfette sollen hier ebenfalls unter funktionelle Flüssigkeiten fallen.

Die in Frage kommenden Schmierstoffe basieren beispielsweise auf mineralischen oder synthetischen Ölen oder Mischungen davon oder auf pflanzlichen und tierischen Ölen, Fetten und Wachsen. Die Schmierstoffe sind dem Fachmann geläufig und in der einschlägigen Fachliteratur, wie beispielsweise in Dieter Klamann, "Schmierstoffe und verwandte Produkte" (Verlag Chemie, Weinheim, 1982), in Schewe-Kobek, "Das Schmiermittel-Taschenbuch" (Dr. Alfred Hüthig-Verlag, Heidelberg, 1974) und in "Ullmanns Enzyklopädie der technischen Chemie", Bd. 13, Seiten 85-94 (Verlag Chemie, Weinheim, 1977) beschrieben.

Die Schmierstoffe sind insbesondere Öle und Fette, beispielsweise basierend auf einem Mineralöl. Bevorzugt sind Öle.

Die Mineralöle basieren insbesondere auf Kohlenwasserstoffverbindungen.

Beispiele von synthetischen Schmierstoffen umfassen Schmierstoffe auf der Basis der aliphatischen oder aromatischen Carboxylester, der polymeren Ester, der Polyalkylenoxide, der Phosphorsäureester, der Poly-α-olefine oder der Silicone, eines Diesters einer zweiwertigen Säure mit einem einwertigen Alkohol, wie z.B. Dioctylsebacat oder Dinonyladipat, eines Triesters von Trimethylolpropan mit einer einwertigen Säure oder mit einem Gemisch solcher Säuren, wie z.B. Trimethylolpropantripelargonat, Trimethylolpropan-tricaprylat oder Gemische davon, eines Tetraesters von Pentaerythrit mit einer einwertigen Säure oder mit einem Gemisch solcher Säuren, wie z.B. Pentaerythrit-tetracaprylat, oder eines komplexen Esters von einwertigen und zweiwertigen Säuren mit mehrwertigen Alkoholen, z.B. ein komplexer Ester von Trimethylolpropan mit Capryl- und Sebacinsäure oder von einem Gemisch davon. Besonders geeignet sind neben Mineralölen z.B. Poly-α-Olefine, Schmierstoffe auf Esterbasis, Phosphate, Glykole, Polyglykole und Polyalkylenglykole, sowie deren Mischungen mit Wasser.

Als pflanzliche Schmierstoffe sind die Öle, Fette und Wachse, gewonnen beispielsweise aus Oliven, Palmen, Palmkernen, Rüben, Raps, Leinen, Nüssen, Soja, Baumwolle, Ricinus, Sonnenblumen, Kürbiskernen, Kokos, Mais oder deren modifizierte Formen, z.B. geschwefelte oder epoxidierte Öle, wie epoxidiertes Sojaöl, sowie Mischungen der Substanzen zu nennen. Beispiele für tierische Öle, Fette und Wachse, die als Schmierstoffe angewandt werden können, sind Talge, Fischöle, Spermöl, Klauenöl, Trane und Specköle, deren modifizierte Formen und Mischungen.

Metallbearbeitungsflüssigkeiten wie Walz-, Zieh- und Schneidöle basieren zumeist auf den oben beschriebenen Mineral- und synthetischen Ölen und können auch als Öl-in-Wasser- bzw. Wasser-in-Öl-Emulsionen vorliegen. Das gleiche gilt für Hydraulikflüssigkeiten. Als weitere funktionelle Flüssigkeiten kommen z.B. Kompressoröle und Spinnpräparationen in Frage.

Die Verbindungen der Formel I, wie oben beschrieben, können z.B. in Mengen von 0.01 bis 10 Gew.-%, zweckmäßig in Mengen von 0.05 bis 5 Gew.-%, vorzugsweise in einer Menge von 0.05 bis 3 Gew.-% und ganz besonders bevorzugt von 0.5 bis 1.5 Gew.-%, bezogen auf die Zusammensetzung, in der funktionellen Flüssigkeit vorliegen.

Die Verbindungen der Formel I können der funktionellen Flüssigkeit auf an sich bekannte Weise beigemischt werden. Die Verbindungen sind beispielsweise in Ölen gut löslich. Es ist auch möglich, einen sogenannten Masterbatch herzustellen, der nach Maßgabe des Verbrauchs auf Einsatzkonzentrationen mit der entsprechenden funktionellen Flüssigkeit verdünnt werden kann.

Zusätzlich zu den erfindungsgemäßen Verbindungen bzw. Mischungen können die erfindungsgemäßen Zusammensetzungen, insbesondere wenn sie organische, vorzugsweise synthetische, Polymere enthalten, noch weitere übliche Additive enthalten. Beispiele für solche Additive sind:
1. Antioxidantien
   1.1. Alkylierte Monophenole, z.B. 2,6-Di-tert-butyl-4-methylphenol, 2-butyl-4,6-dimethylphenol, 2,6-Di-tert-butyl-4-ethylphenol 2,6-Di-tert-butyl-4-n-butylphenol, 2,6-Di-tert-butyl-4-iso-butylphenol, 2,6-Dicyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, 2,6Di-nonyl-4-methylphenol, 2,4-Dimethyl-6-(1'-methyl-undec-1'-yl)-phenol, 2,4-Dimethyl-6-(1'-methyl-heptadec-1'-yl)-phenol, 2,4-Dimethyl-6-(1'-methyl-tridec-1'-yl)-phenol und Mischungen davon.
   1.2. Alkylthiomethylphenole, z.B. 2,4-Di-octylthiomethyl-6-tert-butylphenol, 2,4-Di-octylthiomethyl-6-methylphenol, 2,4-Di-octylthiomethyl-6-ethylphenol, 2,6-Di-dodecylthiomethyl-4-nonylphenol.
   1.3. Hydrochinone und alkylierte Hydrochinone, z.B. 2,6-Di-tert-butyl-4-methoxyphenol, 2,5-Di-tert-butyl-hydrochinon, 2,5-Di-tert-amyl-hydrochinon, 2,6-Diphenyl-4-octadecyl-oxyphenol, 2,6-Di-tert-butyl-hydrochinon, 2,5-Di-tert-butyl-4-hydroxyanisol, 3,5-Di-tert-butyl-4-hydroxyanisol, 3,5-Di-tert-butyl-4-hydroxyphenyl-stearat, Bis-(3,5-di-tert-butyl-4-hydroxyphenyl)adipat.
   1.4. Tocopherole, z.B. α-Tocopherol, β-Tocopherol, γ-Tocopherol, δ-Tocopherol und Mischungen davon (Vitamin E).
   1.5. Hydroxylierte Thiodiphenylether, z.B. 2,2'-Thio-bis-(6-tert-butyl-4-methylphenol), 2,2'-Thio-bis-(4-octylphenol), 4,4'-Thio-bis-(6-tert-butyl-3-methylphenol), 4,4'-Thio-bis-(6-tert-butyl-2-methylphenol), 4,4'-Thio-bis-(3,6-di-sec.-amylphenol), 4,4'-Bis-(2,6-dimethyl-4-hydroxyphenyl)-disulfid.
   1.6. Alkyliden-Bisphenole, z.B. 2,2'-Methylen-bis-(6-tert-butyl-4-methylphenol), 2,2'-Methylen-bis-(6-tert-butyl-4-ethylphenol), 2,2'-Methylen-bis-[4-methyl-6-(α-methyl-cyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert-butylphenol), 2,2'-Ethyliden-bis-(4,6-di-tert-butylphenol), 2,2'-Ethyliden-bis-(6-tert-butyl-4-isobutylphenol), 2,2'-Methylen-bis-[6-(α-methylbenzyl)-4-nonylphenol], 2,2'-Methylen-bis-[6-(α,α-di-methylbenzyl)-4-nonylphenol], 4,4'-Methylen-bis-(2,6-di-tert-butylphenol), 4,4'-Methylen-bis-(6-tert-butyl-2-methylphenol), 1,1-Bis-(5-tert-butyl-4-hydroxy-2-methylphenyl)-butan, 2,6-Bis-(3-tert-butyl-5-methyl-2-hydroxybenzyl)-4-methylphenol, 1,1,3-Tris-(5-tert-butyl-4-hydroxy-2-methylphenyl)-butan, 1,1-Bis-(5-tert-butyl-4-hydroxy-2-methylphenyl)-3-n-dodecylmercaptobutan, Ethylenglycol-bis-[3,3-bis-(3'-tert-butyl-4'-hydroxyphenyl)-butyrat], Bis-(3-tert-butyl-4-hydroxy-5-methyl-phenyl)-dicyclopentadien, Bis-[2-(3'-tert-butyl-2'-hydroxy-5'-methyl-benzyl)-6-tert-butyl-4-methyl-phenyl]-terephthalat, 1,1-Bis-(3,5-dimethyl-2-hydroxyphenyl)-butan, 2,2-Bis-(3,5-di-tert-butyl-4-hydroxyphenyl)-propan, 2,2-Bis-(5-tert-butyl-4-hydroxy-2-methylphenyl)-4-n-dodecylmercaptobutan, 1,1,5,5-Tetra-(5-tert-butyl-4-hydroxy-2-methylphenyl)-pentan.
   1.7. O-, N- und S-Benzylverbindungen, z.B. 3,5,3',5'-Tetra-tert-butyl-4,4'-dihydroxydibenzylether, Octadecyl-4-hydroxy-3,5-dimethylbenzyl-mercaptoacetat, Tris-(3,5-di-tert-butyl-4-hydroxybenzyl)-amin, Bis-(4-tert-butyl-3-hydroxy-2,6-dimethylbenzyl)-dithioterephthalat, Bis-(3,5-di-tert-butyl-4-hydroxybenzyl)-sulfid, Isooctyl-3,5-di-tert-butyl-4-hydroxybenzyl-mercaptoacetat.
   1.8. Hydroxybenzvlierte Malonate, z.B. Dioctadecyl-2,2-bis-(3,5-di-tert-butyl-2-hydroxybenzyl)-malonat, Di-octadecyl-2-(3-tert-butyl-4-hydroxy-5-methylbenzyl)-malonat, Di-dodecylmercaptoethyl-2,2-bis-(3,5-di-tert-butyl-4-hydroxybenzyl)-malonat, Di-[4-(1,1,3,3-tetramethylbutyl)-phenyl]-2,2-bis-(3,5-di-tert-butyl-4-hydroxybenzyl)-malonat.
   1.9. Hydroxybenzyl-Aromaten, z.B. 1,3,5-Tris-(3,5-di-tert-butyl-4-hydroxybenzyl)-2,4,6 trimethylbenzol, 1,4-Bis-(3,5-di-tert-butyl-4-hydroxybenzyl)-2,3,5,6-tetramethylbenzol, 2,4,6-Tris-(3,5-di-tert-butyl-4-hydroxybenzyl)-phenol.
   1.10. Triazinverbindungen, z.B. 2,4-Bis-octylmercapto-6-(3,5-di-tert-butyl-4-hydroxyanilino)-1,3,5-triazin, 2-Octylmercapto-4,6-bis-(3,5-di-tert-butyl-4-hydroxyanilino)-1,3,5-triazin, 2-Octylmercapto-4,6-bis-(3,5-di-tert-butyl-4-hydroxyphenoxy)-1,3,5-triazin, 2,4,6-Tris-(3,5-di-tert-butyl-4-hydroxyphenoxy)-1,2,3-triazin, 1,3,5-Tris-(3,5-di-tert-butyl-4-hydroxybenzyl)-isocyanurat, 1,3,5-Tris-(4-tert-butyl-3-hydroxy-2,6-dimethylbenzyl)-isocyanurat, 2,4,6-Tris-(3,5-di-tert-butyl-4-hydroxyphenylethyl)-1,3,5-triazin, 1,3,5-Tris-(3,5-di-tert-butyl-4-hydroxyphenylpropionyl)-hexahydro-1,3,5-triazin, 1,3,5-Tris-(3,5-dicyclohexyl-4-hydroxybenzyl)-isocyanurat.
   1.11. Benzylphosphonate, z.B. Dimethyl-2,5-di-tert-butyl-4-hydroxybenzylphosphonat, Diethyl-3,5-di-tert-butyl-4-hydroxybenzylphosphonat, Dioctadecyl-3,5-di-tert-butyl-4-hydroxybenzylphosphonat, Dioctadecyl-5-tert-butyl-4-hydroxy-3-methylbenzylphosphonat, Ca-Salz des 3,5-Di-tert-butyl-4-hydroxybenzyl-phosphonsäure-monoethylesters.
   1.12. Acylaminophenole, z.B. 4-Hydroxy-laurinsäureanilid, 4-Hydroxystearinsäureanilid, N-(3,5-di-tert-butyl-4-hydroxyphenyl)-carbaminsäureoctylester.
   1.13. Ester der β-(3,5-Di-tert-butyl-4-hydroxyphenyl)-propionsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo[2.2.2]-octan.
   1.14. Ester der β-(5-tert-Butyl-4-hydroxy-3-methylphenyl)-propionsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   1.15. Ester der β-(3,5-Dicyclohexyl-4-hydroxyphenyl)-propionsäure mit ein- oder mehr wertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythnt, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   1.16. Ester der 3.5-Di-tert-butyl-4-hydroxyphenylessinsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   1.17. Amide der β-(3,5-Di-tert-butyl-4-hydroxyphenyl)-propionsäure, wie z.B. N,N'-Bis- (3,5-di-tert-butyl-4-hydroxyphenylpropionyl)-hexamethylendiamin, N,N'-Bis-(3,5-di-tert-butyl-4-hydroxyphenylpropionyl)-trimethylendiamin, N,N'-Bis-(3,5-di-tert-butyl-4-hydroxyphenylpropionyl)-hydrazin.
2. UV-Absorber und Lichtschutzmittel
   2.1. 2-(2'-Hydroxyphenyl)-benzotriazole, wie z.B. 2-(2'-Hydroxy-5'-methylphenyl)-benzotriazol, 2-(3',5'-Di-tert-butyl-2'-hydroxyphenyl)-benzotriazol, 2-(5'-tert-Butyl-2'-hydroxyphenyl)-benzotriazol, 2-(2'-Hydroxy-5'-(1,1,3,3-tetramethylbutyl)phenyl)-benzotriazol, 2-(3',5'-Di-tert-butyl-2'-hydroxyphenyl)-5-chlor-benzotriazol, 2-(3'-tert-Butyl-2'-hydroxy-5'-methylphenyl)-5-chlor-benzotriazol, 2-(3'-sec-Butyl-5'-tert-butyl-2'-hydroxyphenyl)-benzotriazol, 2-(2'-Hydroxy-4'-octoxyphenyl)-benzotriazol, 2-(3',5'-Di-tert-amyl-2'-hydroxyphenyl)-benzotriazol, 2-(3',5'-Bis-(α,α-dimethylbenzyl)-2'-hydroxyphenyl)-benzotriazol, Mischung aus 2-(3'-tert-Butyl-2'-hydroxy-5'-(2-octyloxycarbonylethyl)-phenyl)-5-chlor-benzotriazol, 2-(3'-tert-Butyl-5'-[2-(2-ethylhexyloxy)-carbonylethyl]-2'-hydroxyphenyl)-5-chlor-benzotriazol, 2-(3'-tert-Butyl-2'-hydroxy-5'-(2-methoxycarbonylethyl)phenyl)-5-chlor-benzotriazol, 2-(3'-tert-Butyl-2'-hydroxy-5'-(2-methoxycarbonylethyl)phenyl)-benzotriazol, 2-(3'-tert-Butyl-2'-hydroxy-5'-(2-octyloxycarbonylethyl)phenyl)-benzotriazol, 2-(3'-tert-Butyl-5'-[2-(2-ethylhexyloxy)carbonylethyl]-2'-hydroxyphenyl)-benzotriazol, 2-(3'-Dodecyl-2'-hydroxy-5'-methylphenyl)-benzotriazol, und 2-(3'-tert-Butyl-2'-hydroxy-5'-(2-isooctyloxycarbonylethyl)phenyl-benzotriazol, 2,2'-Methylen-bis[4-(1,1,3,3-tetramethylbutyl)-6-benzotriazol-2-yl-phenol]; Umesterungsprodukt von 2-[3'-tert-Butyl-5'-(2-methoxycarbonylethyl)-2'-hydroxy-phenyl]-benzotriazol mit Polyethylenglycol 300; [R-CH₂CH₂-COO(CH₂)₃ 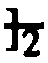 mit R = 3'-tert-Butyl-4'-hydroxy-5'-2H-benzotriazol-2-yl-phenyl.
   2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.
   2.3. Ester von gegebenenfalls substituierten Benzoesäuren, wie z.B. 4-tert-Butyl-phenylsalicylat, Phenylsalicylat, Octylphenyl-salicylat, Dibenzoylresorcin, Bis-(4-tert-butyl-benzoyl)-resorcin, Benzoylresorcin, 3,5-Di-tert-butyl-4-hydroxybenzoesäure-2,4-di-tert-butylphenylester, 3,5-Di-tert-butyl-4-hydroxybenzoesäurehexadecylester, 3,5-Di-tert-butyl-4-hydroxybenzoesäure-octadecylester, 3,5-Di-tert-butyl-4-hydroxybenzoesäure-2-methyl-4,6-di-tert-butylphenylester.
   2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.
   2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert-butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.
   2.6. Sterisch gehinderte Amine, wie z.B. Bis-(2,2,6,6-tetramethyl-piperidyl)-sebacat, Bis-(2,2,6,6-tetramethyl-piperidyl)-succinat, Bis-(1,2,2,6,6-pentamethylpiperidyl)-sebacat, n-Butyl-3,5-di-tert-butyl-4-hydroxybenzyl-malonsäure-bis(1,2, 2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis-(2,2,6,6-Tetramethyl-4-piperidyl)-hexamethylendiamin und 4-tert-Octylamino-2,6-dichlor-1,3,5-s-triazin, Tris-(2,2,6,6-tetramethyl- 4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethyl-piperazinon), 4-Benzoyl-2,2,6,6-tetramethylpiperidin, 4-Stearyloxy-2,2,6,6-tetramethylpiperidin, Bis-(1 ,2,2,6,6-pentamethylpiperidyl)-2-n-butyl-2-(2-hydroxy-3,5-di-tert-butylbenzyl)-malonat, 3-n-Octyl-7,7,9,9-tetramethyl- 1 ,3,8-triazaspiro[4.5]decan-2,4-dion, Bis-(1-octyloxy-2,2,6,6-tetramethylpiperidyl)-sebacat, Bis-(1-octyloxy-2,2,6,6-tetramethylpiperidyl)-succinat, Kondensationsprodukt aus N,N'-Bis-(2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-n-butylamino-2,2,6,6-tetramethylpiperidyl)-1,3,5-triazin und 1,2-Bis-(3-aminopropylamino)äthan, Kondensationsprodukt aus 2-Chlor-4,6-di-(4-n-butylamino-1,2,2,6,6-pentamethylpiperidyl)-1,3,5-triazin und 1,2-Bis-(3-aminopropylamino)-äthan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6-pentamethyl-4-piperidyl)-pyrrolidin-2,5-dion.
   2.7. Oxalsäurediamide, wie z.B. 4,4'-Di-octyloxy-oxanilid, 2,2'-Diethoxy-oxanilid, 2,2'-Di-octyloxy-5,5'-di-tert-butyl-oxanilid, 2,2'-Di-dodecyloxy-5,5'di-tert-butyloxanilid, 2-Ethoxy-2'-ethyl-oxanid, N,N'-Bis-(3-dimethylaminopropyl)-oxalamid, 2-Ethoxy-5-tert-butyl-2'-ethyloxanilid und dessen Gemisch mit 2-Ethoxy-2'-ethyl-5,4'-di-tert-butyl-oxanilid, Gemische von o- und p-Methoxy- sowie von o- und p-Ethoxy-di-substituierten Oxaniliden.
   2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis-(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-[2-hydroxy-4-(2-hydroxy-3-butyloxy-propyloxy)phenyl]4,6-bis(2,4-dimethylphenyl)-1,3,5-triazin, 2-[2-hydroxy-4-(2-hydroxy-3-octyloxy-propyloxy)phenyl]-4,6-bis(2,4-dimethylphenyl)-1-triazin.
3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert-butyl-4-hydroxyphenylpropionyl)-hydrazin, 3-Salicyloylamino-1,2,4-triazol, Bis-(benzyliden)oxalsäuredihydrazid, Oxanilid, Isophthalsäure-dihydrazid, Sebacinsäure-bis-phenylhydrazid, N,N'-Diacetyl-adipinsäure-dihydrazid, N,N'-Bis-salicyloyl-oxalsäure-dihydrazid, N,N'-Bis-salicyloyl-thiopropionsäure-dihydrazid.
4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert-butylphenyl)-phosphit, Diisodecylpentaerythrit-diphosphit, Bis-(2,4-di-tert-butylphenyl)-pentaerythritdiphosphit, Bis-(2,6-di-tert-butyl-4-methylphenyl)-pentaerythritdiphosphit, Bis-isodecyloxy-pentaerythritdiphosphit, Bis-(2,4-di-tert-butyl-6-methylphenyl)-pentaerythritdiphosphit, Bis-(2,4,6-tri-tert-butylphenyl)-pentaerythritdiphosphit, Tristearyl-sorbit-triphosphit, Tetrakis-(2,4-di-tert-butylphenyl)-4,4'-biphenylen-diphosphonit, 6-Isooctyloxy-2,4,8,10-tetra-tert-butyl-12H-dibenz[d,g]-1,3,2-dioxaphosphocin, 6-Fluor-2,4,8,10-tetra-tert-butyl-12-methyl-di-benz[d,g]-1,3,2-dioxaphosphocin, Bis-(2,4-di-tert-butyl-6-methylphenyl)-methylphosphit, Bis-(2,4-di-tert-butyl-6-methylphenyl)-ethylphosphit.
5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.
6. Polyamidstabilisatoren, wie z.B. Kupfersalze in Kombination mit Jodiden und/oder Phosphorverbindungen und Salze des zweiwertigen Mangans.
7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Behenat, Mg-Stearat, Na-Ricinoleat, K-Palmitat, Antimonbrenzcatechinat oder Zinnbrenzcatechinat.
8. Nukleierungsmittel, wie z.B. 4-tert-Butylbenzoesäure, Adipinsäure, Diphenylessigsäure.
9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Ruß, Graphit.
10. Sonstige Zusätze, wie z.B. Weichmacher, Gleitmittel, Emulgatoren, Pigmente, Optische Aufheller, Flammschutzmittel, Antistatika, Treibmittel.
11. Benzofuranone bzw. Indolinone, wie z.B. in US-A-4 325 863, US-A-4 338 244, US-A-5 175 312, US-A-5 216 052, US-A-5 252 643, DE-A-4 316 611, DE-A-4 316 622, DE-A-4 316 876, EP-A-0 589 839 oder EP-A-0 591 102 beschrieben, oder 3-[4-(2-Acetoxyethoxy)phenyl]-5,7-di-tert-butyl-benzofuran-2-on, 5,7-Di-tert-butyl-3-[4-(2-stearoyloxyethoxy)phenyl] -benzofuran-2-on, 3,3'-Bis-[5,7-di-tert-butyl-3-(4-[2-hydroxyethoxy]-phenyl)-benzofuran-2-on], 5,7-Di-tert-butyl-3-(4-ethoxyphenyl)benzofuran-2-on, 3-(4-Acetoxy-3,5-dimethylphenyl)-5,7-di-tert-butyl-benzofuran-2-on, 3-(3,5-Dimethyl-4-pivaloyloxy-phenyl)-5,7-di-tert-butyl-benzofuran-2-on.

Handelt es sich bei den erfindungsgemäßen Zusammensetzungen um solche auf Basis von funktionellen Flüssigkeiten, insbesondere Schmierstoffen und Hydraulikflüssigkeiten bzw. Metallbearbeitungsflüssigkeiten, können diese ebenfalls weitere Additive enthalten, die zugegeben werden, um gewisse Gebrauchseigenschaften zu verbessern, wie z.B. weitere Antioxidantien, Metalldesaktivatoren, Rostinhibitoren, Viskositäts-Index-Verbesserer, Stockpunkterniedriger, Dispergiermittel/Tenside und Verschleißschutzadditive. Beispiele hierfür sind:

### Beispiele für phenolische Antioxidantien:

Solche können den vorstehenden Punkten 1.1. bis 1.17 entnommen werden.

### Beispiele für aminische Antioxidantien:

N,N'-Di-isopropyl-p-phenylendiamin, N,N'-Di-sec-butyl-p-phenylendiamin, N,N'-Bis-(1,4-dimethyl-pentyl)-p-phenylendiamin, N,N'-Bis(1-ethyl-3-methyl-pentyl)-p-phenylendiamin, N,N'-Bis(1-methyl-heptyl)-p-phenylendiamin, N,N'-Dicyclohexyl-p-phenylendiamin, N,N'-Diphenyl-p-phenylendiamin, N,N'-Di-(naphthyl-2)-p-phenylendiamin, N-Isopropyl-N'-phenyl-p-phenylendiamin, N-(1,3-Dimethyl-butyl)-N'-phenyl-p-phenylendiamin, N-(1-Methyl-heptyl)-N'-phenyl-p-phenylendiamin, N-Cyclohexyl-N'-phenyl-p-phenylendiamin, 4-(p-Toluol-sulfonamido)-diphenylamin, N,N'-Dimethyl-N,N'-di-sec-butyl-p-phenylendiamin, Diphenylamin, N-Allyldiphenylamin, 4-Isopropoxy-diphenylamin, N-Phenyl-1-naphthylamin, N-Phenyl-2-naphthylamin, octyliertes Diphenylamin, z.B. p,p'-Di-tert-octyldiphenylamin, 4-n-Butylaminophenol, 4-Butyrylamino-phenol, 4-Nonanoylamino-phenol, 4-Dodecanoylamino-phenol, 4-Octadecanoylaminophenol, Di-(4-methoxyphenyl)-amin, 2,6-Di-tert-butyl-4-dimethylamino-methyl-phenol, 2,4'-Diamino-diphenylmethan, 4,4'-Diamino-diphenylmethan, N,N,N',N'-Tetramethyl-4,4'-diamino-diphenylmethan, 1,2-Di-[(2-methyl-phenyl)-amino]-ethan, 1,2-Di-(phenylamino)-propan, (o-Tolyl)-biguanid, Di-[4-(1',3'-dimethyl-butyl)-phenyl]amin, tert-octyliertes N-Phenyl-1-naphthylamin, Gemisch aus mono- und dialkylierten tert-Butyl/tert-Octyldiphenylaminen, Gemisch aus mono- und dialkylierten Isopropyl/Isohexyl-diphenylaminen, Gemische aus mono- und dialkylierten tert-Butyldiphenylaminen, 2,3-Dihydro-3,3-dimethyl-4H-1,4-benzothiazin, Phenothiazin, N-Allylphenothiazin, N,N,N',N'-Tetraphenyl-1,4-diamino-but-2-en, N,N-Bis-(2,2,6,6-tetramethyl-piperidin-4-yl-hexamethylendiamin, Bis-(2,2,6,6-tetramethylpiperidin-4-yl)-sebacat,2,2,6,6-Tetramethylpiperidin-4-on, 2,2,6,6-Tetramethylpiperidin-4-ol.

### Beispiele für weitere Antioxidantien:

Aliphatische oder aromatische Phosphite, Ester der Thiodipropionsäure oder der Thiodiessigsäure, oder Salze der Dithiocarbamid- oder Dithiophosphorsäure, 2,2,12,12-Tetramethyl-5,9-dihydroxy-3,7,11-trithiatridecan und 2,2,15,15-Tetramethyl-5,12-dihydroxy-3,7,10, 14-tetrathiahexadecan.

### Beispiele für Metall-Desaktivatoren, z.B. für Kupfer, sind:

a) Benzotriazole und deren Derivate, z.B. 4- oder 5-Alkylbenztriazole (z.B. Tolutriazol) und deren Derivate, 4,5,6,7-Tetrahydrobenztriazol, 5,5'-Methylenbis-benztriazol; Mannich-Basen von Benztriazol oder Tolutriazol wie 1-[Di(2-ethylhexyl)aminomethyl)-tolutriazol und 1-[Di(2-ethylhexyl)aminomethyl)-benztriazol; Alkoxyalkylbenztriazole wie 1-(Nonyloxymethyl)-benztriazol, 1-(1-Butoxyethyl)-benztriazol und 1-(1-Cyclohexyloxybutyl)-tolutriazol.
b) 1,2,4-Triazole und deren Derivate, z.B. 3-Alkyl (oder Aryl)-1,2,4-Triazole, Mannich-Basen von 1,2,4-Triazolen wie 1-[Di(2-ethylhexyl)aminomethyl- 1,2,4-triazol; Alkoxyalkyl-1,2,4-triazole wie 1-(1-Butoxyethyl-1,2,4-triazol; acylierte 3-Amino-1,2,4-triazole.
c) Imidazolderivate, z.B. 4,4'-Methylenbis(2-undecyl-5-methylimidazol, Bis[(N-methyl)imidazol-2-yl]carbinol-octylether.
d) Schwefelhaltige heterocyclische Verbindungen, z.B. 2-Mercaptobenzthiazol, 2,5-Di-mercapto-1,3,4-thiadiazol und deren Derivate; 3,5-Bis[di(2-ethylhexyl)amino-methyl]-1,3,4-thiadiazolin-2-on.
e) Aminoverbindungen, z.B. Salicyliden-propylendiamin, Salicylaminoguanidin und deren Salze.

### Beispiele für Rost-Inhibitoren sind:

a) Organische Säuren, ihre Ester, Metallsalze, Aminsalze und Anhydride, z.B. Alkyl- und Alkenylbernsteinsäuren und deren Partialester mit Alkoholen, Diolen oder Hydroxycarbonsäuren, Partialamide von Alkyl- und Alkenylbernsteinsäuren, 4-Nonylphenoxyessigsäure, Alkoxy- und Alkoxyethoxycarbonsäuren wie Dodecyloxyessigsäure, Dodecyloxy(ethoxy)-essigsäure und deren Aminsalze, ferner N-Oleoyl-sarcosin, Sorbitan-mono-oleat, Blei-naphthenat, Alkenylbernsteinsäureanhydride, z.B. Dodecenylbernsteinsäure-anhydrid, 2-Carboxymethyl-1-dodecyl-3-methylglycerin und dessen Aminsalze.
b) Stickstoffhaltige Verbindungen, z.B.:
   I. Primäre, sekundäre oder tertiäre aliphatische oder cycloaliphatische Amine und Aminsalze von organischen und anorganischen Säuren, z.B. öllösliche Alkylammoniumcarboxylate, ferner 1-[N,N-bis-(2-hydroxyethyl)amino]-3-(4-nonylphenoxy)-propan-2-ol.
   II. Heterocyclische Verbindungen, z.B.:
      Substituierte Imidazoline und Oxazoline, 2-Heptadecenyl-1-(2-hydroxyethyl)-imidazolin.
c) Phosphorhaltige Verbindungen, z.B.:
   Aminsalze von Phosphorsäurepartialestern oder Phosphonsäurepartialestern, Zinkdialkyldithiophosphate.
d) Schwefelhaltige Verbindungen, z.B.:
   Barium-dinonylnaphthalin-sulfonate, Calciumpetroleum-sulfonate, Alkylthio-substituierte aliphatische Carbonsäuren, Ester von aliphatischen 2-Sulfocarbonsäuren und deren Salze.
e) Glycerinderivate, z.B.:
   Glycerin-monooleat, 1-(Alkylphenoxy)-3-(2-hydroxyethyl)glycenne, 1-(Alkylphenoxy)-3-(2,3-dihydroxypropyl)glycerine, 2-Carboxyalkyl-1,3-dialkylglycerine.

### Beispiele für Viskositätsindex-Verbesserer sind:

Polyacrylate, Polymethacrylate, Vinylpyrrolidon/Methacrylat-Copolymere, Polyvinylpyrrolidone, Polybutene, Olefin-Copolymere, Styrol/Acrylat-Copolymere, Polyether.

### Beispiele für Stockpunkterniedriger sind:

Polymethacrylat, alkylierte Naphthalinderivate.

### Beispiele für Dispergiermittel/Tenside sind:

Polybutenylbernsteinsäureamide oder -imide, Polybutenylphosphonsäurederivate, basische Magnesium-, Calcium-, und Bariumsulfonate und -phenolate.

### Beispiele für Verschleissschutz-Additive sind:

Schwefel und/oder Phosphor und/oder Halogen enthaltende Verbindungen, wie geschwefelte Olefine und pflanzliche Oele, Zinkdialkyldithiophosphate, alkylierte Triphenylphosphate, Tritolylphosphat, Tricresylphosphat, chlorierte Paraffine, Alkyl- und Aryldi- und tri-sulfide, Aminsalze von Mono- und Dialkylphosphaten, Aminsalze der Methylphosphonsäure, Diethanolaminomethyltolyltriazol, Di(2-ethylhexyl)aminomethyltolyltriazol, Derivate des 2,5-Dimercapto-1,3,4-thiadiazols, 3-[(Bis-isopropyloxy-phosphinothioyl)-thiol-propionsäure-ethylester, Triphenylthiophosphat (Triphenylphosphorothioat), Tris-(alkylphenyl)phosphorothioate und deren Gemische, (z.B. Tris(isononylphenyl)phosphorothioat), Diphenylmonononylphenyl-phosphorothioat, Isobutylphenyl-diphenylphosphorothioat, Dodecylaminsalz des 3-Hydroxy-1,3-thiaphosphetan-3-oxids, Trithiophosphorsäure-5,5,5-tris[isooctylacetat (2)], Derivate von 2-Mercaptobenzthiazol wie 1-[N,N-Bis(2-ethylhexyl)aminomethyl-2-mercapto-1H-1,3-benzthiazol, Ethoxycarbonyl-5-octyl-dithiocarbamat.

Die vorliegende Erfindung betrifft auch die Verwendung von Verbindungen der Formel I zum Stabilisieren von gegen oxidativen, thermischen und/oder lichtinduzierten Abbau empfindlichem organischem Material, insbesondere von natürlichen oder (halb)synthetischen Polymeren oder von funktionellen Flüssigkeiten, vor allem von Schmierstoffen. Die Verbindungen wirken beispielsweise besonders gut als Antioxidantien und Ablagerungsverhinderer (deposit control agents) in funktionellen Flüssigkeiten, wie oben erwähnt.

Bevorzugte Verbindungen der Formel I, wie vorstehend beschrieben, führen zu bevorzugten Zusammensetzungen.

Ein Vorteil der Verbindungen der Formel I ist, daß z.B. bei der Anwendung in Schmierstoffen sowohl Antioxidans- als auch Ablagerungsverhinderungs-(Deposit Control)-wirkung erzielt wird.

Da es oftmals nötig ist, einem Substrat mehrere Zusatzstoffe beizufügen, können Löslichkeitsprobleme auftreten. Dies betrifft insbesondere Anwendungen in Ölen und flüssigen Polymeren. Auch diesbezüglich zeigen die erfindungsgemäßen Verbindungen gute Eigenschaften.

Die Verbindungen der Formel I werden beispielsweise nach folgendem Schema hergestellt:

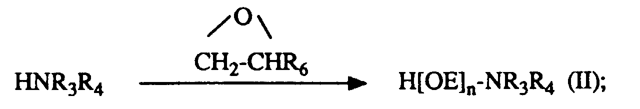

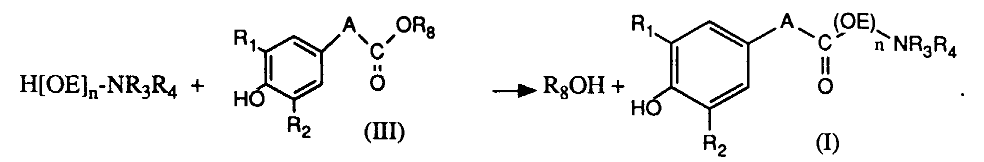

Dabei stehen
R₁, R₂, A, E, n, R₃, R₄, und R₆ für die bereits oben nach der Formel I beschriebenen Reste und
R₈ für H oder Niederalkyl, z.B. C₁-C₄-Alkyl, besonders Methyl.

Die Verbindungen der Formel I können z.B. durch Veresterung bzw. Umesterung einer 4-Hydroxyphenylpropion- oder essigsäure bzw. deren Ester der Formel III mit einer Verbindung der Formel II, z.B. katalysiert mit einer Brφnsted-Säure bzw. einer Lewis-Säure hergestellt werden [vgl. die folgenden Beispiele 1-4]. Als Katalysatoren eignen sich neben Brφnsted-Säuren und Lewis-Säuren auch Aluminiumsilikate, Ionenaustauscherharze, Zeolithe, natürlich vorkommende Schichtsilikate ("saure Erden" bzw. "acid clays", wie z.B. Fuller's Earth) oder modifizierte Schichtsilikate.

Geeignete Brφnsted-Säuren sind Mineralsäuren oder organische Säuren. Als anorganische Säuren kommen Halogenwasserstoffsäuren und Sauerstoffsäuren, insbesondere des Schwefels und des Phosphors in Frage. Beispiele sind Salzsäure, Schwefelsäure, Phosphorsäure. Als organische Säuren gelten Carbonsäuren und organische Reste tragende Sulfo- und Phospho-Gruppen enthaltende Säuren. Beispiele sind Methansulfonsäure, p-Toluolsulfonsäure, Essig- und Propionsäure. Besonders bevorzugt ist die p-Toluolsulfonsäure.

Geeignete Lewis-Säuren sind beispielsweise Zinntetrachlorid, Aluminiumchlorid, Zinkchlorid, Dibutylzinnoxid oder Bortrifluoriddiethyletherat. Dibutylzinnoxid ist bevorzugt.

Die Herstellung der (Di)arylaminoalkohole der Formel II erfolgt zweckmäßig über die Addition der entsprechenden Alkylenoxide an die (Di)arylamine bei von der Reaktivität abhängigen Temperaturen, gegebenenfalls unter Kühlung, vorzugsweise jedoch zwischen 0 und 60, insbesondere zwischen 15 und 40°C bzw. um Raumtemperatur, wenn nötig unter Druck, vorzugsweise in Anwesenheit von stöchiometrischen Mengen Base, z.B. aus der Gruppe der Alkalimetalle und Alkalimetallorganyle, wie Natrium oder Butyllithium [beschrieben in G.A. Epling, A. Kumar, Synlett 1991/5, 347-8] oder säurekatalysiert, z.B. mit den oben erwähnten Lewis-Säuren wie Zinkchlorid oder Bortrifluoriddiethyletherat [beschrieben in DE 1,543,335].

Die Erfindung betrifft somit auch ein Verfahren zur Herstellung von Verbindungen der Formel I, dadurch gekennzeichnet, daß in einem ersten Schritt (Di)arylamine der Formel HNR₃R₄ mit Epoxiden der Formel 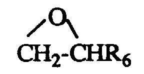 umgesetzt werden und
in einem zweiten Schritt die hydroxyalkylierten Zwischenprodukte

H[OE]ₙ-NR₃R₄ (II)

mit Verbindungen der Formel 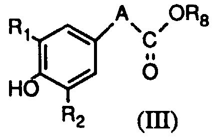 umgesetzt werden,
wobei A, E, R₁, R₂, R₃, R₄ und R₆ die bereits anfangs nach Formel I beschriebenen Bedeutungen haben, und R₈ für H oder C₁-C₄-Alkyl steht.

Bei der Durchführung des ersten Verfahrensschrittes leitet man beispielsweise das Epoxid in das Reaktionsgefäß ein oder gibt es zu und setzt zweckmäßig solange um, bis vom Arylamin kaum noch etwas übrig ist. Währenddessen hat sich aber gewöhnlich bereits ein Anteil an höher hydroxyalkyliertem (hauptsächlich n = 2) (Di)arylaminoalkohol II gebildet. Wird die Reaktion im geschlossenen System (Autoklav) durchgeführt, so kann der Anteil der Verbindungen mit n > 1 abhängig vom eingesetzten Überschuß an Epoxid höher werden.

Gegenstand der Erfindung sind daher auch Mischungen von Verbindungen der Formel I mit verschiedenem Index n.

Insgesamt reagiert demzufolge im ersten Schritt je nach Umsatz meist ein molarer Überschuß an Epoxid. Die genaue Menge an benötigtem Epoxid hängt von der Reaktionsführung ab. Möglich sind Batch-Betrieb, beispielsweise im Autoklaven oder bei Normaldruck mit Einleitung des Epoxids, oder kontiniuerlicher Betrieb, ggf. mit Rückführung des unumgesetzten Epoxids.
Auch beim zweiten Schritt des Verfahrens ist im allgemeinen ein geringer molarer Überschuß an Alkohol zweckmäßig, z.B. bis zu 20 mol%, insbesondere bis zu 10 mol%, bezogen auf die Hydroxyphenylcarbonsäure der Formel III bzw. deren Ester.

Bei der Verwendung von Epoxiden 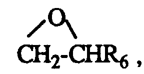 bei denen R₆ nicht für Wasserstoff steht (R₆ ≠ H) ist zu beachten, daß die Orientierung der Gruppe -E- im (Di)arylaminoalkohol II sowie im Produkt I zu zwei verschiedenen Produkten führt (E ist, wie anfangs bereits beschrieben, -CHR₆-CH₂- oder -CH₂CHR₆-). Die zweite Möglichkeit ist gewöhnlich weitaus überwiegend, und entsprechend ist die erste Variante in den folgenden Beispielen nicht getrennt aufgeführt, ist aber in den entsprechenden Produkten vorhanden.

Schritt 1 kann mit und ohne Lösungsmittel durchgeführt werden. Bevorzugt arbeitet man in einem polaren aprotischen Lösungsmittel. Beispiele sind Tetrahydrofuran (THF), Dimethylformamid (DMF), Dimethylacetamid (DMA), Hexamethylphosphorsäuretrisamid (HMPTA), Dimethylsulfoxid (DMSO) und Tetramethylharnstoff (TMU), ferner Pyridin oder Alkylpyridine. Besonders bevorzugt als Lösungsmittel ist wasserfreies DMF.

Bei Schritt 2 kann ebenfalls ohne Lösungs- oder Verdünnungsmittel gearbeitet werden. Die Verwendung eines bei Reaktionsbedingungen inerten Lösungsmittels ist jedoch bevorzugt. Das Lösungsmittel ist zweckmäßig dasselbe wie das später bei der Reinigung verwendete. Bevorzugt sind Kohlenwasserstoffe, besonders bevorzugt Toluol. Das Lösungsmittel wird besonders auch dazu benutzt, den entstehenden Alkohol azeotrop abzudestillieren.

Die Temperatur liegt bei Schritt 1 zweckmäßig bei 0 bis 60°C, bevorzugt bei 15 bis 40°C. Bei Verwendung von reaktiveren Aminen, insbesondere einfach aromatischen, kann es zweckmäßig sein, zu kühlen. Der Druck kann gegebenenfalls verändert werden, liegt aber bevorzugt im Atmosphärenbereich. Bei Schritt 2 kann gegebenenfalls der entstehende Alkohol unter verringertem Druck abgezogen werden, und die Temperatur kann beispielsweise bei 70 bis 180°C, bevorzugt bei 120 bis 160°C liegen.

Die bei der Herstellung der Verbindungen entstehenden Gemische mit verschiedenem Index n bzw. mit verschiedenen Orientierungen der Gruppe E können gewöhnlich ohne weitere Reinigungsschritte ihrer Verwendung zugeführt werden. Selbstverständlich ist aber eine Trennung mit gängigen Methoden wie Chromatographie, Destillation etc. möglich, wenn die reinen Verbindungen erhalten werden sollen.

Die folgenden Beispiele erläutern die Erfindung weiter, ohne sie jedoch zu beschränken. Teile- und Prozentangaben beziehen sich, sofern nicht anders angegeben, auf das Gewicht. "t-Bu" bedeutet tert-Butyl.

### Beispiel 1

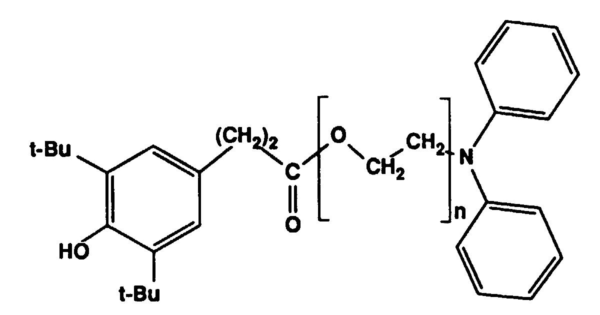

n= 1,2,3

Zu einer Lösung von 52.3 g (0.31 mol) Diphenylamin in 600 ml wasserfreiem Dimethylformamid gibt man 8.3 g (0.36 mol) metallisches Natrium. Nach 20 min bei 90°C ist das Natrium umgesetzt, und es wird auf Raumtemperatur abgekühlt. In die braune Lösung wird bei 15 - 25 °C langsam Ethylenoxid eingeleitet, bis dünnschichtchromatographisch fast kein Diphenylamin mehr nachzuweisen ist (ca. 2 h). Nach dem Abkühlen auf 10°C wird mit ca. 190 ml 2 N HCl auf pH 6 angesäuert. Nach Extraktion mit Toluol, Waschen der Toluolphase mit Wasser und Eindampfen erhält man 61.8 g viskoses, braunes Öl, das direkt weiterverwendet wird.

Zu einer Mischung von 30 g dieses Zwischenproduktes und 36.7 g (0.126 mol) 3-(3,5-di-tert-butyl-4-hydroxyphenyl)-propionsäuremethylester (CA Reg. No. 6386-38-5) gibt man 1.4 g Dibutylzinnoxid. Aus der klaren Schmelze destilliert man bei 140°C das Methanol langsam ab. 110 ml Toluol werden binnen 6 h bei 140°C zugetropft und sofort zusammen mit etwaigem restlichen Methanol abdestilliert.

Das braune Reaktionsprodukt wird in 100 ml Hexan klar gelöst und diese Lösung durch 30 g Silikagel filtriert (Toluol/Hexan 1:1 bis Toluol) und eingedampft. Man erhält 57.0 g klares, gelbes, viskoses Öl: n_{D}²⁰ = 1.5726; ca. 95% Ausbeute bezogen auf 3-(3,5-di-tert-butyl-4-hydroxyphenyl)-propionsäuremethylester.

Das Produkt besteht laut GC-Analyse aus 63% n=1, 23% n=2, 2.1% n=3, 5.7% Diphenylamin und 2.6% 3-(3,5-di-tert-butyl-4-hydroxyphenyl)-propionsäuremethylester.

### Beispiel 2

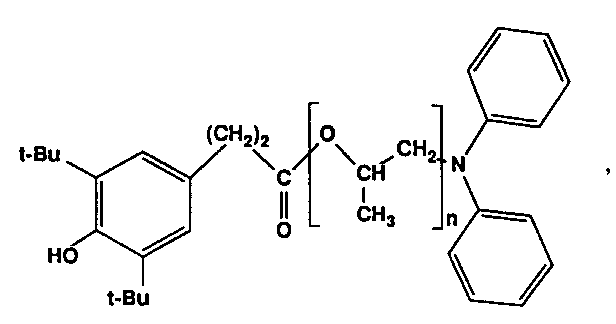

n = 1,2,3

Zu einer Lösung von 17.4 g (0.10 mol) Diphenylamin in 100 ml wasserfreiem Dimethylformamid gibt man 2.5 g (0.11 mol) metallisches Natrium. Nach 30 min bei 80°C ist das Natrium umgesetzt, und es wird auf Raumtemperatur abgekühlt. Nach Zugabe von 9.8 ml Propylenoxid steigt die Temperatur auf 31°C, und es wird noch 3 h bei 40°C weitergerührt. Nach dem Abkühlen auf 10°C wird mit 50 ml 2 N HCl auf pH 6 angesäuert. Nach Extraktion mit Toluol, Waschen der Toluolphase mit Wasser und Eindampfen erhält man 22.4 g klares gelbes mittelviskoses Öl, das direkt weiterverwendet wird.

Zu einer Mischung von 22.0 g dieses Zwischenproduktes und 26.0 g (0.089 mol) 3-(3,5-di-tert-butyl-4-hydroxyphenyl)-propionsäuremethylester (CA Reg. No. 6386-38-5) gibt man 1.1 g Dibutylzinnoxid. 50 ml Toluol werden binnen 5 h bei 140°C zugetropft und sofort zusammen mit dem entstehenden Methanol abdestilliert.

Das braune, mittelviskose Reaktionsprodukt wird mit etwas Toluol verdünnt, durch 20 g Silikagel filtriert (Toluol) und eingedampft. Man erhält 42 g klares, schwach hellgelbes, viskoses Öl: n_{D}²⁰ 1.5650; ca. 97% Ausbeute, bezogen auf 3-(3,5-di-tert-butyl-4-hydroxyphenyl)-propionsäuremethylester.
Das Produkt besteht laut GC-Analyse aus 77.0% n=1, 14.6% n=2, <1% n=3, 2.6% Diphenylamin und 3.0 % 3-(35-di-tert-butyl-4-hydroxyphenyl)-propionsäuremethylester.

In gleicher Weise wie unter Beispiel 2 beschrieben, werden *mutatis mutandis* die Verbindungen der Beispiele 3 und 4 hergestellt.

### Beispiel 3

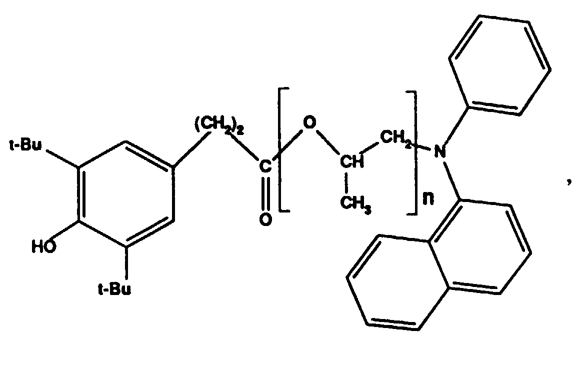

n= 1,2

Ausbeute: 95%, bezogen auf 3-(3,5-di-tert-butyl-4-hydroxyphenyl)-propionsäuremethylester.
Eigenschaften: klares, hochviskoses, hellbraunes Öl; das Produkt besteht laut GC-Analyse aus 76.6% n=1, 14.9% n=2, 3.0% Phenyl-α-naphthylamin und 3.0 % 3-(3,5-di-tert-butyl-4-hydroxyphenyl)-propionsäuremethylester.

### Beispiel 4

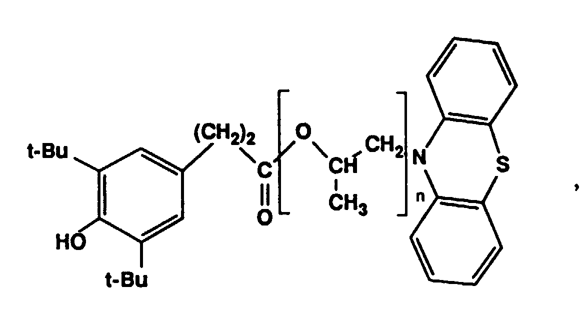

n= 1,2
Ausbeute: 90%, bezogen auf 3-(3,5-di-tert-butyl-4-hydroxyphenyl)-propionsäuremethylester
Zusammensetzung: Laut DC (Silikagel/Toluol und Silikagel/Dichlormethan) > 95% n=1.
Eigenschaften: kristallisiertes Produkt, Smp. 130-131°C.

### Beispiel 5

Deposit and Oxidation Panel Test (DOPT)

Es handelt sich bei diesem Test um eine Variante einer Prüfmethode für Motoröle, insbesondere für Dieselmotoröle, welche von G. Abellaneda et al., IIIe Symposium CEC, 1989, New Cavendish Street. London WIM8AR, England, beschrieben wurde. Er dient dazu, die Eignung der Öle mit dem jeweiligen Stabilisator zur Verhinderung von Ablagerungen am Kolben zu prüfen.

Dabei wird in oxidierender Atmosphäre mit definierter Geschwindigkeit Öl auf eine schrägstehende heiße Metallplatte (Panel) getropft und so ein Ölfilm erzeugt. Die Testdauer beträgt 20 Stunden, die Temperatur auf der Metallplatte 260°C, der Luftdurchsatz 9.7 l/h und die Fließgeschwindigkeit des Öls 1 ml/min. Die feuchte Luft-Atmosphäre wird mit 460 ppm NO₂ und 25 ppm SO₂ angereichert.

Nach dem Test wird die Metallplatte zur Entfernung des Öls in Petrolether getaucht, getrocknet, gewogen und visuell bewertet, z.B. daraufhin, ob sich ein Lack gebildet hat. Je kleiner Gewicht und visuelle Bewertungszahl sind, desto besser ist das Ergebnis. Als Schmieröl wird handelsübliches Öl der API-Klassifikation CD, welches mit dem Grundöl STANCO 150 verdünnt wird, verwendet.

Diesem vorbereiteten Öl werden die in Tabelle I angegebenen Stabilisatoren in einer Menge von 0.6 Gew.%, bezogen auf das Öl, zugemischt und der DOPT Prüfung unterzogen.

**Tabelle I:**

| Deposit and Oxidation Panel Test | | | |
|---|---|---|---|
| Produkt gemäß Beispiel | Konzentration [Gew %] | Ablagerung | |
| | | Gewicht [mg] | visuell |
| 2 | 0.6 | 6 | 2 |
| 4 | 0.6 | 4 | 0 |
| ohne Zusatz | - | 72 | 14 |

## Patentansprüche

1. Verbindungen der Formel I 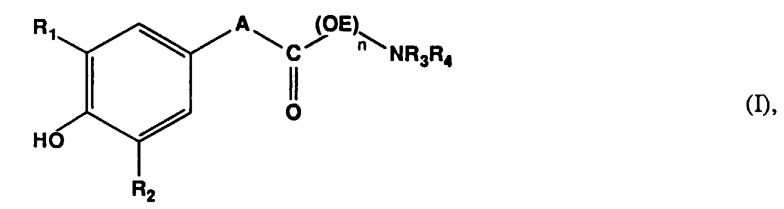 worin
R₁ und R₂ unabhängig voneinander C₁-C₂₀-Alkyl, Allyl, Methallyl, unsubstituiertes oder mit C₁-C₈-Alkyl substituiertes C₅-C₁₂-Cycloalkyl, C₅-C₈-Cycloalkenyl, Phenyl oder C₇-C₉-Phenylalkyl bedeuten,
A für eine direkte Bindung, -CH₂-, -(CH₂)₂- oder -CH₂-CH(CH₃)- steht,
E -(CH₂)₂- , -CHR₆-CH₂- oder -CH₂CHR₆- darstellt,
n 1,2 oder 3 ist,
R₃ C₁-C₁₈-Alkyl oder durch -O- oder -S- unterbrochenem C₂-C₁₈-Alkyl , unsubstituiertem oder mit C₁-C₈-Alkyl substituiertem C₅-C₁₂-Cycloalkyl, Phenyl, Naphthyl, C₇-C₉-Phenylalkyl, C₇-C₁₈-Alkylphenyl oder der Formel 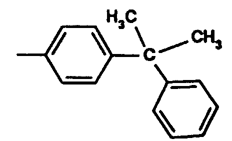 entspricht,
R₄ Phenyl, Naphthyl, C₇-C₉-Phenylalkyl, C₇-C₁₈-Alkylphenyl oder ein Rest der Formel 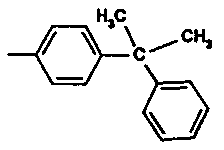 ist,
oder
NR₃R₄ eine Gruppe der Formel 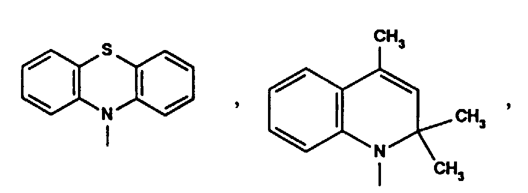 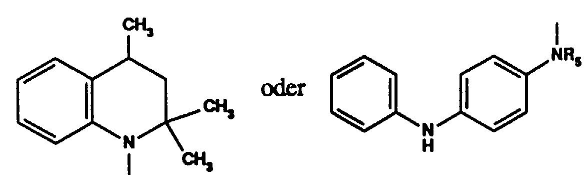 bedeutet,
R₅ für Wasserstoff, C₁-C₁₈-Alkyl, Allyl, Methallyl, unsubstituiertes oder mit C₁-C₈-Alkyl substituiertes C₅-C₁₂-Cycloalkyl, Phenyl oder Naphthyl steht,
R₆ Wasserstoff oder C₁-C₂₀-Alkyl oder durch -O-, -S-, -NR₇- oder -C(O)O- unterbrochenes C₂-C₂₀-Alkyl ist, und
R₇ Wasserstoff oder C₁-C₆-Alkyl darstellt.

2. Verbindungen der Formel I gemäß Anspruch 1, worin
R₁ und R₂ unabhängig voneinander C₁-C₈-Alkyl, C₅-C₈-Cycloalkyl oder C₇-C₉-Phenylalkyl bedeuten,
A für -(CH₂)₂- oder -CH₂-CH(CH₃)- steht,
n 1 oder 2 ist,
R₃ und R₄ unabhängig voneinander
Phenyl, Naphthyl, C₇-C₉-Phenylalkyl, C₇-C₁₈-Alkylphenyl entsprechen, oder
NR₃R₄ eine Gruppe der Formel 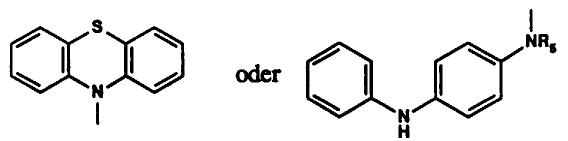 bedeutet,
R₅ Wasserstoff, C₁-C₈-Alkyl oder Phenyl ist und
R₆ Wasserstoff, C₁-C₂₀-Alkyl oder durch -O- oder -S- unterbrochenes C₂-C₂₀-Alkyl ist.

3. Verbindungen nach Anspruch 2, worin
R₁ und R₂ unabhängig voneinander C₁-C₅-Alkyl, C₅-C₆-Cycloalkyl oder C₇-C₉-Phenylalkyl bedeuten,
n 1 ist,
R₃ und R₄ unabhängig voneinander
Phenyl, Naphthyl, C₇-C₉-Phenylalkyl oder C₇-C₁₈-Alkylphenyl sind, oder
NR₃R₄ eine Gruppe der Formel 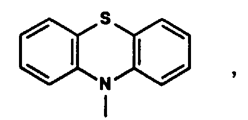 bedeutet, und
R₆ Wasserstoff oder C₁-C₄-Alkyl ist.

4. Verbindungen gemäß Anspruch 1 der Formel 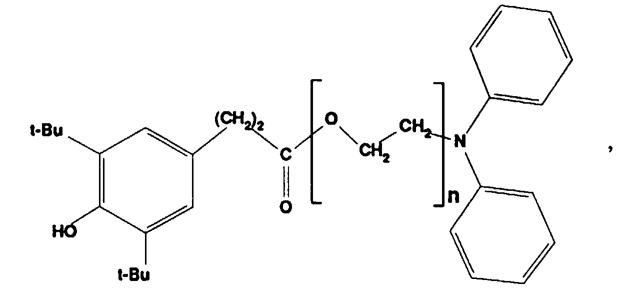 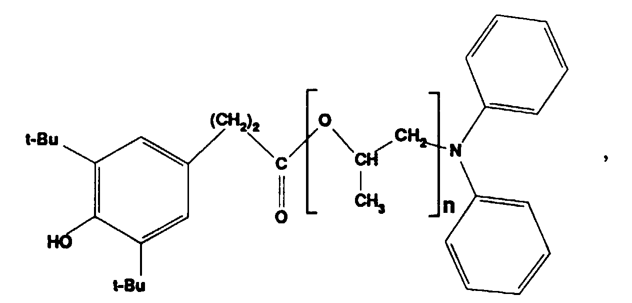 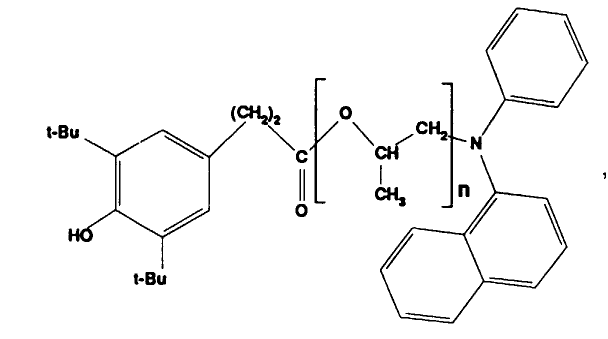 oder 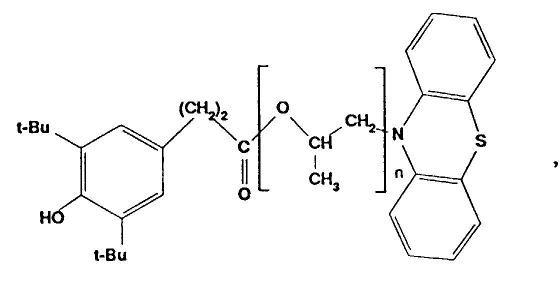 wobei n für 1, 2 und 3 stehen kann und t-Bu die Abkürzung von tert-Butyl ist.

5. Verfahren zur Herstellung von Verbindungen der Formel I gemäß Anspruch 1, dadurch gekennzeichnet, daß in einem ersten Schritt (Di)arylamine der Formel HNR₃R₄ mit Epoxiden der Formel 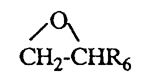 umgesetzt werden und in einem zweiten Schritt die hydroxyalkylierten Zwischenprodukte
H[OE]ₙ-NR₃R₄ (II)
mit Verbindungen der Formel 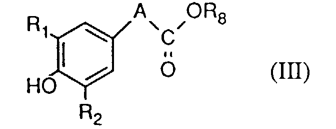 umgesetzt werden, wobei A, E, n, R₁, R₂, R₃, R₄ und R₆ die in Anspruch 1 beschriebenen Bedeutungen haben, und R₈ Wasserstoff oder C₁-C₄-Alkyl ist.

6. Verfahren gemäß Anspruch **5**, dadurch gekennzeichnet, daß beim ersten Schritt mit einer Base aus der Gruppe der Alkalimetalle und Alkalimetallorganyle katalysiert wird.

7. Zusammensetzungen enthaltend
A) ein gegen lichtinduzierten, thermischen oder /und oxidativen Abbau empfindliches organisches Material und
B) mindestens eine Verbindung der Formel I gemäß Anspruch 1.

8. Zusammensetzungen nach Anspruch **7**, worin das organische Material ein natürliches, halbsynthetisches oder synthetisches Polymer oder eine funktionelle Flüssigkeit ist.

9. Zusammensetzungen nach Anspruch **8**, worin die funktionelle Flüssigkeit ein Schmierstoff oder eine Hydraulikflüssigkeit ist.

10. Verwendung von Verbindungen der Formel I gemäß Anspruch **1** zum Stabilisieren von gegen oxidativen, thermischen und/oder lichtinduzierten Abbau empfindlichem organischen Material.

11. Verwendung nach Anspruch **10** zum Stabilisieren von natürlichen, halbsynthetischen oder synthetischen Polymeren oder von funktionellen Flüssigkeiten.

12. Verwendung nach Anspruch **11** zum Stabilisieren von Schmierstoffen oder Hydraulikflüssigkeiten.

13. Verfahren zum Stabilisieren von organischem Material gegen oxidativen, thermischen und/oder lichtinduzierten Abbau, dadurch gekennzeichnet, daß man diesem Material als Stabilisatoren Verbindungen der Formel I gemäß Anspruch **1** zusetzt bzw. auf dieses aufbringt.

14. Verfahren nach Anspruch **13** zum Stabilisieren von natürlichen, halbsynthetischen oder synthetischen Polymeren oder von funktionellen Flüssigkeiten.

15. Verfahren nach Anspruch **14** zum Stabilisieren von synthetischen Polymeren oder von Schmierstoffen oder Hydraulikflüssigkeiten.

## Claims

1. A compound of the formula I 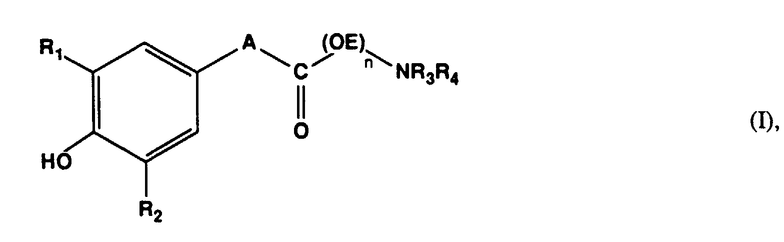 in which
R₁ and R₂ are independently of one another C₁-C₂₀alkyl, allyl, methallyl, unsubstituted or
C₁-C₈alkyl-substituted C₅-C₁₂cycloalkyl, C₅-C₈cycloalkenyl, phenyl or C₇-C₉phenylalkyl,
A is a direct bond, -CH₂-, -(CH₂)₂- or -CH₂-CH(CH₃)-,
E is -(CH₂)₂-, -CHR₆-CH₂- or -CH₂CHR₆-,
n is 1, 2 or 3,
R₃ is C₁-C₁₈alkyl or is C₂-C₁₈alkyl which is interrupted by -O- or -S-, unsubstituted or
C₁-C₈alkyl-substituted C₅-C₁₂cycloalkyl, phenyl, naphthyl, C₇-C₉phenylalkyl,
C₇-C₁₈alkylphenyl or is of the formula 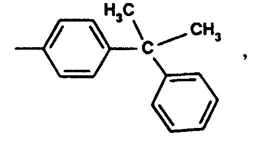
R₄ is phenyl, naphthyl, C₇-C₉phenylalkyl, C₇-C₁₈alkylphenyl or is a radical of the formula 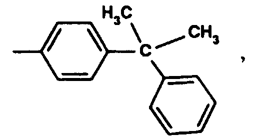 or
NR₃R₄ is a group of the formula 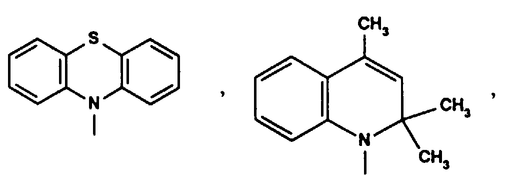 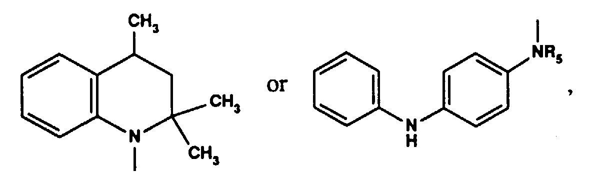
R₅ is hydrogen, C₁-C₁₈alkyl, allyl, methallyl, unsubstituted or C₁-C₈alkyl-substituted C₅-C₁₂cycloalkyl, phenyl or naphthyl,
R₆ is hydrogen, C₁-C₂₀alkyl or C₂-C₂₀alkyl which is interrupted by -O-, -S-, -NR₇- or -C(O)O-, and
R₇ is hydrogen or C₁-C₆alkyl.

2. A compound of the formula I according to claim 1, in which
R₁ and R₂ are independently of one another C₁-C₈alkyl, C₅-C₈cycloalkyl or
C₇-C₉phenylalkyl,
A is -(CH₂)₂- or -CH₂-CH(CH₃)-,
n is 1 or 2,
R₃ and R₄ are independently of one another phenyl, naphthyl, C₇-C₉phenylalkyl or
C₇-C₁₈alkylphenyl, or
NR₃R₄ is a group of the formula 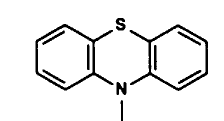 or 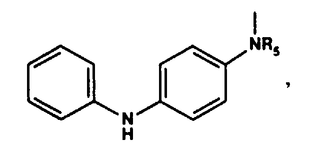
R₅ is hydrogen, C₁-C₈alkyl or phenyl, and
R₆ is hydrogen, C₁-C₂₀alkyl or C₂-C₂₀alkyl which is interrupted by -O- or -S-.

3. A compound according to claim 2, in which
R₁ and R₂ are independently of one another C₁-C₅alkyl, C₅-C₆cycloalkyl or
C₇-C₉phenylalkyl,
n is 1,
R₃ and R₄ are independently of one another phenyl, naphthyl, C₇-C₉phenylalkyl or
C₇-C₁₈alkylphenyl, or
NR₃R₄ is a group of the formula 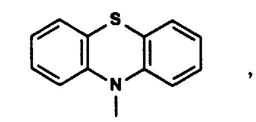 and
R₆ is hydrogen or C₁-C₄alkyl.

4. A compound according to claim 1 of the formula 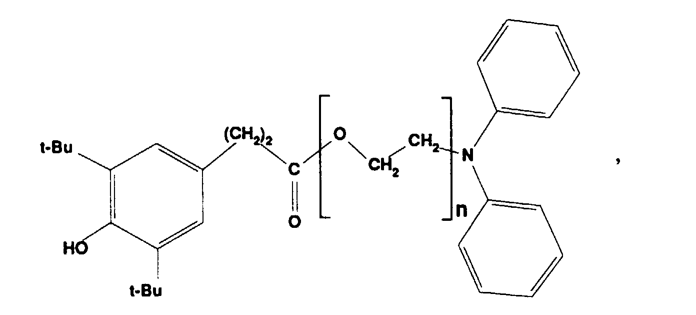 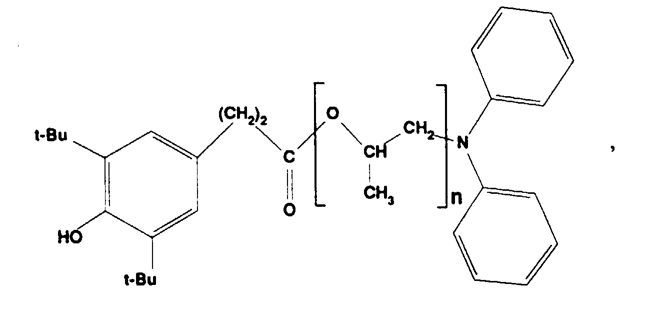 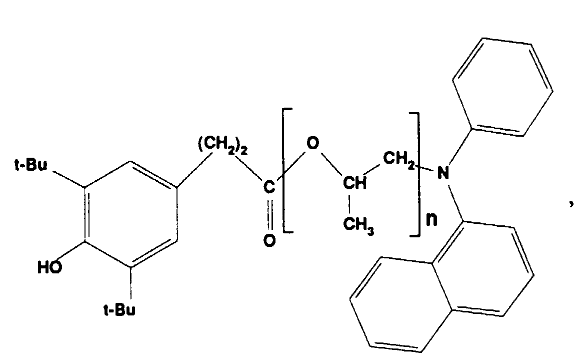 or 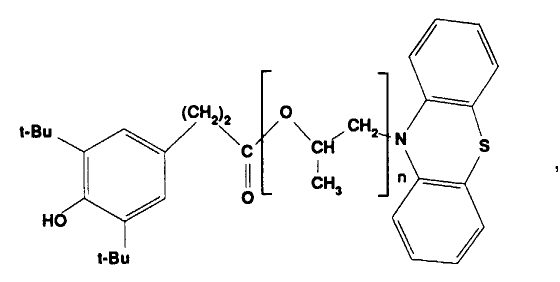 in which n is 1, 2 or 3 and t-Bu is the abbreviation of tert-butyl.

5. A process for the preparation of compounds of the formula I according to claim 1, which comprises in a first step reacting (di)arylamines of the formula HNR₃R₄ with epoxides of the formula 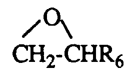 and in a second step reacting the hydroxyalkylated intermediates
H[OE]ₙ-NR₃R₄ (II)
with compounds of the formula 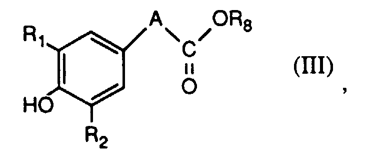 in which A, E, n, R₁, R₂, R₃, R₄ and R₆ are as described in claim 1, and R₈ is hydrogen or C₁-C₄alkyl.

6. A process according to claim 5, wherein the first step is catalysed with a base from the group consisting of the alkali metals and alkali metal organyls.

7. A composition comprising
A) an organic material which is sensitive to light-induced, thermal and/or oxidative degradation, and
B) at least one compound of the formula I according to claim 1.

8. A composition according to claim 7, in which the organic material is a natural, semisynthetic or synthetic polymer or a functional fluid.

9. A composition according to claim 8, in which the functional fluid is a lubricant or a hydraulic fluid.

10. Use of a compound of the formula I according to claim 1 for stabilizing organic material which is sensitive to oxidative, thermal and/or light-induced degradation.

11. Use according to claim 10 for the stabilization of natural, semisynthetic or synthetic polymers or functional fluids.

12. Use according to claim 11 for the stabilization of lubricants or hydraulic fluids.

13. Process for stabilizing organic material against oxidative, thermal and/or light-induced degradation, which comprises adding or applying to this material, as stabilizers, compounds of the formula I according to claim 1.

14. A process according to claim 13 for the stabilization of natural, semisynthetic or synthetic polymers or functional fluids.

15. A process according to claim 14 for the stabilization of synthetic polymers, lubricants or hydraulic fluids.

## Revendications

1. Composé de formule I 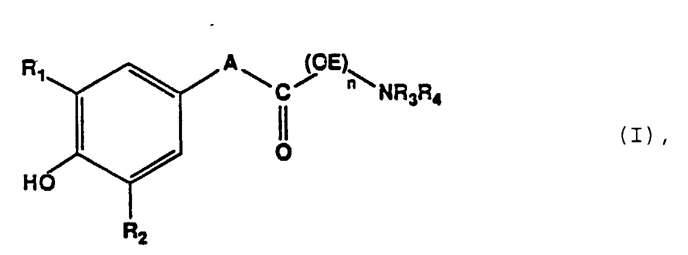 dans laquelle
R₁ et R₂, indépendamment l'un de l'autre, représentent des groupes alkyle en C₁-C₂₀, allyle, méthallyle, cycloalkyle en C₅-C₁₂ non substitué ou cycloalkyle en C₅-C₁₂ substitué par un groupe alkyle en C₁-C₈, cycloalcényle en C₅-C₈, phényle ou phénylalkyle en C₇-C₉,
A représente une liaison directe, -CH₂- -(CH₂)₂- ou -CH₂-CH(CH₃)-,
E représente -(CH₂)₂-, -CHR₆-CH₂- ou -CH₂CHR₆-,
n vaut 1, 2 ou 3,
R₃ représente un groupe alkyle en C₁-C₁₈ ou un groupe alkyle en C₂-C₁₈ interrompu par O ou S, cycloalkyle en C₅-C₁₂ non substitué ou cycloalkyle en C₅-C₁₂ substitué par un groupe alkyle en C₁-C₈, phényle, naphtyle, phénylalkyle en C₇-C₉, (alkyl en C₇-C₁₈)-phényle ou répond à la formule 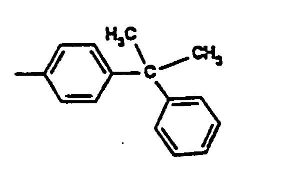
R₄ représente un groupe phényle, naphtyle, phénylalkyle en C₇-C₉, (alkyl en C₇-C₁₈)-phényle, ou un reste de formule 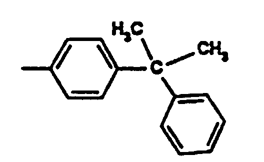
ou
NR₃R₄ représente un groupe de formule 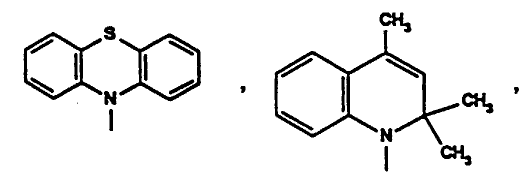 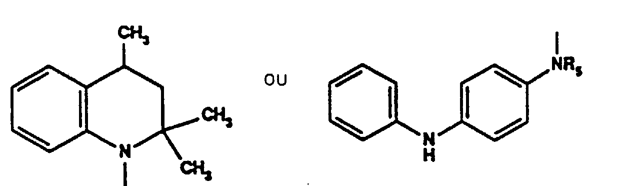
R₅ représente un atome d'hydrogène, des groupes alkyle en C₁-C₁₈, allyle, méthallyle, cycloalkyle en C₅-C₁₂ non substitué ou substitué par un groupe alkyle en C₁-C₈, phényle ou naphtyle,
R₆ représente un atome d'hydrogène, alkyle en C₁-C₂₀, ou alkyle en C₂-C₂₀ interrompu par -O-, -S-, -NR- ou -C(O)O-, et
R₇ représente un atome d'hydrogène ou un groupe alkyle en C₁-C₆.

2. Composé de formule I selonla revendication 1, dans laquelle
R₁ et R₂, indépendamment l'un de l'autre, représentent un groupe alkyle en C₁-C₈, cycloalkyle en C₅-C₈ ou phénylalkyle en C₇-C₉,
A représente -(CH₂)₂- ou -CH₂-CH(CH₃)-,
n vaut 1 ou 2,
R₃ et R₄, indépendamment l'un de l'autre, représentent des groupes phényle, naphtyle, phénylalkyle en C₇-C₉, (alkyl en C₇-C₁₈)phényle,
ou
NR₃R₄ représente un groupe de formule 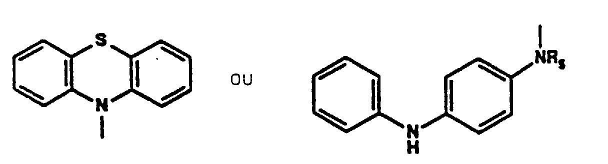
R₅ représente un atome d'hydrogène, un groupe alkyle en C₁-C₈ ou phényle, et
R₆ représente un atome d'hydrogène, un groupe alkyle en C₁-C₂₀ ou un groupe alkyle en C₂-C₂₀ interrompu par -O- ou -S-.

3. Composés selon la revendication 2, dans lesquels
R₁ et R₂, indépendamment l'un de l'autre, représentent un groupe alkyle en C₁-C₅, cycloalkyle en C₅-C₆ ou phénylalkyle en C₇-C₉,
n vaut 1,
R₃ et R₄, indépendamment l'un de l'autre, représentent des groupes phényle, naphtyle, phénylalkyle en C₇-C₉, (alkyl en C₇-C₁₈)phényle,
ou
NR₃R₄ représente un groupe de formule 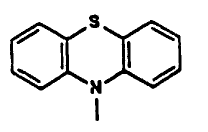 et
R₆ représente un groupe alkyle en C₁-C₄.

4. Composés selon la revendication 1, de formules 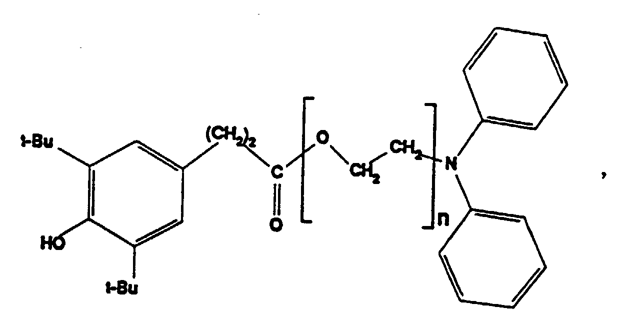 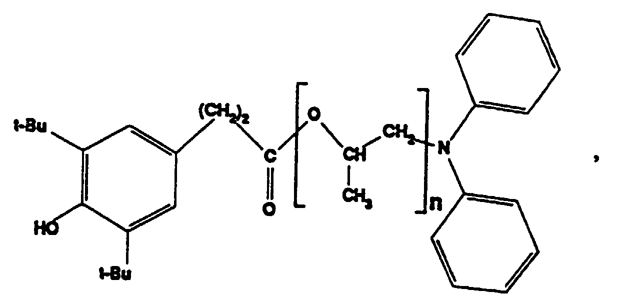 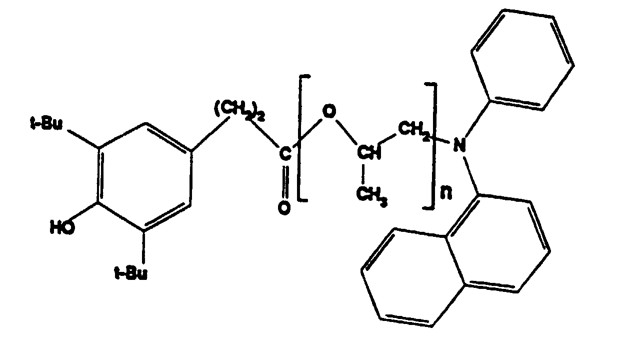 ou 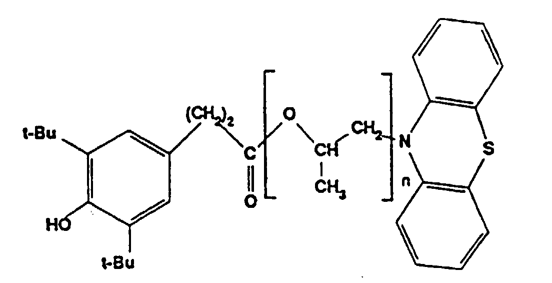 n pouvant valoir 1, 2 et 3, et l'abréviation t-Bu représente tert-butyle.

5. Procédé pour la préparation de composés de formule I selon la revendication 1, caractérisé en ce que dans une première étape on fait réagir des (di)arylamines de formule HNR₃R₄ avec des époxydes de formule 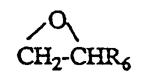 et dans une deuxième étape on fait réagir les produits intermédiaires hydroxyalkylés H[OE]ₙ-NR₃R₄ (II) avec des composés de formule 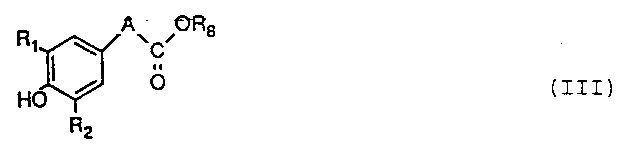 où A, E, n, R₁, R₂, R₃, R₄ et R₆ ont les significations déjà données dans la formule I et R₈ représente un atome d'hydrogène ou un groupe alkyle en C₁-C₄.

6. Procédé selon la revendication 5, caractérisé en ce que, dans une première étape, on effectue la catalyse à l'aide d'une base prise dans le groupe des métaux alcalins et des composés organométalliques des métaux alcalins.

7. Composition contenant
A) une matière organique sensible à la dégradation induite par la lumière, par la chaleur et/ou par l'oxygène, et
B) au moins un composé de formule I selon la revendication 1.

8. Composition selon la revendication 7, dans laquelle la matière organique est un polymère naturel, semi-synthétique ou synthétique ou un fluide fonctionnel.

9. Composition selon la revendication 8, dans laquelle le fluide fonctionnel est un lubrifiant ou un fluide hydraulique.

10. Utilisation de composés de formule I selon la revendication 1 pour la stabilisation de matières organiques sensibles à la dégradation induite par l'oxygène, la chaleur et/ou la lumière.

11. Utilisation selon la revendication 10 pour la stabilisation de polymères naturels, semi-synthétiques ou synthétiques ou de fluides fonctionnels.

12. Utilisation selon la revendication 11 pour la stabilisation de lubrifiants ou de fluides hydrauliques.

13. Procédé pour la stabilisation de matières organiques contre la dégradation induite par l'oxygène, la chaleur et/ou la lumière, caractérisé en ce que l'on ajoute, respectivement on pose sur ces matières en tant que stabilisants des composés de formule I selon la revendication 1.

14. Procédé selon la revendication 13, pour la stabilisation de polymères naturels, semi-synthétiques ou synthétiques, ou de fluides fonctionnels.

15. Procédé selon la revendication 14, pour la stabilisation de polymères synthétiques ou de lubrifiants ou de fluides hydrauliques.
